# EUROPEAN PATENT APPLICATION

(11) **EP 4 595 974 A2**
(43) Date of publication of application: **06.08.2025**
(21) Application number: 25161427.7
(22) Date of filing: 22.10.2019
(51) Int. Cl.: A61K 38/18

(54) **THERAPEUTIC COMBINATIONS OF TDFRPS AND ADDITIONAL AGENTS AND METHODS OF USE**

(30) Priority: 22.10.2018 US 201862748636 P
(62) Divisional of application: 19875690.0
(71) Applicant: Carlson, William D., Weston, MA 02493 (US)
(72) Inventor: Carlson, William D., Weston, MA 02493 (US)
(74) Representative: Marks & Clerk LLP

(57) **Abstract**

The present disclosure relates generally to methods of treating or preventing a tissue differentiation factor-associated disorder or disease and methods of treating or preventing fibrosis in a subject using tissue differentiation factor related polypeptide (TDFRPs) in combination with an additional agent. The present disclosure also relates to compositions and pharmaceutical compositions comprising the TDFRPs and an additional agent.

## Description

### RELATED APPLICATIONS

This Application claims the benefit of and priority to US Provisional Application No. 62/748,636 filed October 22, 2018, the contents of which is expressly incorporated herein by reference in its entirety.

### BACKGROUND OF THE INVENTION

Cell differentiation is the central characteristic of tissue morphogenesis, which initiates during embryogenesis, and continues to various degrees throughout the life of an organism in adult tissue repair and regeneration mechanisms. The degree of morphogenesis in adult tissue varies among different tissues and is related, among other things, to the degree of cell turnover in a given tissue.

Morphogenic polypeptides are capable of inducing the developmental cascade of cellular and molecular events that culminate in the formation of new organ-specific tissue, including any vascularization, connective tissue formation, and nerve innervation as required by the naturally occurring tissue. The polypeptides have been shown to induce morphogenesis of cartilage and bone, as well as, periodontal tissues, dentin, liver, heart, kidney and neural tissue, including retinal tissue.

These tissue morphogenic polypeptides are a distinct subfamily of polypeptides different from other members of the TGF-beta superfamily in that they share a high degree of sequence identity in the C-terminal domain and in that the tissue morphogenic polypeptides are able to induce, on their own, the full cascade of events that result in formation of functional tissue rather than merely inducing formation of fibrotic (scar) tissue. Specifically, members of the family of morphogenic polypeptides are capable of all of the following in a morphogenetically permissive environment: stimulating cell proliferation and cell differentiation, and supporting the growth and maintenance of differentiated cells. The morphogenic polypeptides also may act as endocrine, paracrine or autocrine factors. As a result of their biological activities, significant effort has been directed toward the development of morphogen-based therapeutics for treating injured or diseased mammalian tissue.

Fibrotic diseases are characterized by the activation of fibroblasts, increased production of collagen and fibronectin, and transdifferentiation into contractile myofibroblasts. This process usually occurs over many months and years, and can lead to organ dysfunction or death. Examples of fibrotic diseases include diabetic nephropathy, liver cirrhosis, idiopathic pulmonary fibrosis, rheumatoid arthritis, atherosclerosis, cardiac fibrosis and scleroderma (systemic sclerosis; SSc). Fibrotic disease represents one of the largest groups of disorders for which there is no effective therapy and thus represents a major unmet medical need. Often the only redress for patients with fibrosis is organ transplantation; since the supply of organs is insufficient to meet the demand, patients often die while waiting to receive suitable organs. Lung fibrosis alone can be a major cause of death in scleroderma lung disease, idiopathic pulmonary fibrosis, radiation- and chemotherapy-induced lung fibrosis and in conditions caused by occupational inhalation of dust particles. The lack of appropriate antifibrotic therapies arises primarily because the etiology of fibrotic disease is unknown.

Pro-fibrotic proteins such as transforming growth factor-beta (TGF-β) and connective tissue growth factor (CTGF) have been implicated to involve in fibrotic diseases. As TGF-β induces fibroblasts to synthesize and contract ECM, this cytokine has long been believed to be a central mediator of the fibrotic response (1). CTGF, discovered more than a decade ago as a protein secreted by human endothelial cells (2), is induced by TGF-β and is considered a downstream mediator of the effects of TGF-β on fibroblasts ( 3, 4). Similarly, TGF-β induces expression of the ED-A form of the matrix protein fibronectin (ED-A FN), a variant of fibronectin that occurs through alternative splicing of the fibronectin transcript (5). This induction of ED-A FN is required for TGF-β1-triggered enhancement of α-SMA and collagen type I expression (6). Thus TGF-β has been implicated as a "master switch" in induction of fibrosis in many tissues including lung (7) and kidney (ref). In this regard, TGF-β is upregulated in lungs of patients with IPF, or in kidneys of CKD patients and expression of active TGF-β in lungs or kidneys of rats induces a dramatic fibrotic response, whereas the inability to respond to TGF-β1 affords protection from bleomycin-induced fibrosis (8) or renal interstitial fibrosis (30).

Epithelial to mesenchymal transition (EMT), a process whereby fully differentiated epithelial cells undergo transition to a mesenchymal phenotype giving rise to fibroblasts and myofibroblasts, is increasingly recognized as playing an important role in repair and scar formation following epithelial injury. The extent to which this process contributes to fibrosis following injury in the lung and other organs is a subject of active investigation. Recently, it was demonstrated that transforming growth factor (TGF)-β induces EMT in alveolar epithelial cells (AEC) in vitro and in vivo, and epithelial and mesenchymal markers have been colocalized to hyperplastic type II (AT2) cells in lung tissue from patients with idiopathic pulmonary fibrosis (IPF), suggesting that AEC may exhibit extreme plasticity and serve as a source of fibroblasts and/or myofibroblasts in lung fibrosis. TGF-β1 was first described as an inducer of EMT in normal mammary epithelial cells (9) and has since been shown to mediate EMT in vitro in a number of different epithelial cells, including renal proximal tubular, lens, and most recently alveolar epithelial cells (10-14).

Modulation of the TGF-β-dependent Smad pathway in animal models has provided strong evidence for a role for TGF-β in fibrotic EMT in vivo. EMT of lens epithelial cells in vivo following injury is completely prevented in Smad3 null mice, while primary cultures of Smad3_/_ lens epithelial cells treated with TGF-β are protected from EMT (15). Similarly, in the kidney, Smad3 null mice are protected from experimentally induced tubulointerstitial fibrosis and show reduced EMT and collagen accumulation, whereas cultures of renal tubular epithelial cells from Smad3_/_ animals show a block in EMT and a reduction in autoinduction of TGF-β1 (16). In human proximal tubular epithelial cells, increased CTGF and decreased E-cadherin were Smad3-dependent, increased MMP-2 was Smad2-dependent, and increases in α-SMA were dependent on both (17). A recent transcriptomic analysis of TGF-β-induced EMT in normal mouse and human epithelial cells demonstrated, using a dominant negative approach, that Smad signaling was critical for regulation of all tested target genes (18). Non-Smad-dependent pathways implicated in TGF-β-dependent EMT include RhoA, Ras, p38 MAPK, PI3 kinase, Notch, and Wnt signaling pathways. In most cases, stimulation of these co-operative pathways provides the context for induction and specification of EMT within a particular tissue, with Smads representing the dominant pathway, which in some instances may be necessary but not sufficient for induction of full EMT (19).

A number of interventions have been demonstrated to lead to the reversal of EMT. BMP-7 reversed TGF-β1-induced EMT in adult tubular epithelial cells by directly counteracting TGF-β-induced Smad3-dependent EMT, and evidence for reversal of renal fibrosis occurring via EMT has been shown in vivo (20). BMP-7 was able to delay EMT in lens epithelium in association with downregulation of Smad2, whereas overexpression of inhibitory Smad7 prevented EMT and decreased nuclear translocation of Smads2 and -3 (21). EMT is ameliorated in Smad3 knockout mice (15, 16), and Smad7, an antagonist of TGF-β signaling, or bone morphogenetic protein-7 (BMP-7) acting in a Smad-dependent manner, can reverse or delay fibrosis in renal and lens epithelia (21, 22). Furthermore, HGF blocks EMT in human kidney epithelial cells by upregulation of the Smad transcriptional co-repressor SnoN, which leads to formation of a transcriptionally inactive SnoN/Smad complex, thereby blocking the effects of TGF-β1 (23). Knowledge of the precise molecular mechanisms mediating TGF-β-induced EMT and its interactions with other signaling pathways will be important for developing strategies to inhibit/reverse EMT without disrupting the beneficial effects of TGF-β signaling.

The development of new treatment strategies for treating and preventing tissue differentiation factor-associated disorders or treating or preventing fibrosis is important. Thus, a need remains in the art to develop appropriate therapeutic approaches that will reverse or stop the progression of disease.

### SUMMARY OF THE INVENTION

According to one aspect, the disclosure features a method of treating or preventing a tissue differentiation factor-associated disorder or disease, the method comprising administering to a subject in need of treatment at least one tissue differentiation factor related polypeptide (TDFRP) in combination with an additional agent, wherein the additional agent is selected from the group consisting of an inhibitor of angiotensin converting enzyme (ACE), a neprilysin inhibitor and an angiotensin receptor-neprilysin inhibitor, wherein the TDFRP and additional agent are administered in an amount effective to treat or prevent the tissue differentiation factor-associated disorder or disease in the subject.

According to one embodiment, the serum half-life of the TFDRP is increased when administered with the additional agent, compared to administration of the TDFRP alone. According to one embodiment, the serum half-life is increased 2-fold or more when the TDFRP is administered with the additional agent. According to one embodiment, the serum half-life is increased 5-fold or more when the TDFRP is administered with the additional agent. According to one embodiment, the serum half-life is increased 10-fold or more when the TDFRP is administered with the additional agent. According to one embodiment, the tissue differentiation factor-associated disorder is selected from the group consisting of a tissue degenerative disease and tissue regeneration disease. According to another embodiment, the tissue regeneration disease or disorder is a disease or disorder of the kidney or renal tissue. According to another embodiment, the disease or disorder of the kidney or renal tissue is selected from the group consisting of acute kidney injury and chronic kidney disease. According to one embodiment, treating the disease or disorder of the kidney comprises restoring the function of the kidney. According to another embodiment, the tissue regeneration disease or disorder is a disease or disorder of the heart or cardiovascular system. According to one embodiment, treating the disease or disorder of the heart or cardiovascular system comprises restoring the function of the heart or cardiovascular system. According to another embodiment, the disease or disorder of the heart of cardiovascular system is selected from the group consisting of pulmonary artery hypertension, acute myocardial infarction, chronic congestive heart failure, cardiomyopathy and coronary vasculopathy. According to another embodiment, the tissue regeneration disease or disorder is a disease or disorder of the liver or hepatic system. According to another embodiment, the disease or disorder of the liver or hepatic system is liver failure. According to one embodiment, treating the disease or disorder of the kidney comprises restoring the function of the liver or hepatic system. According to another embodiment, the tissue regeneration disease or disorder is cancer. According to one embodiment, the cancer is breast cancer. According to one embodiment, the cancer is prostate cancer. According to one embodiment, the cancer is a glioblastoma. According to one embodiment, the cancer is a glioblastoma. According to one embodiment, the tissue degenerative disease is selected from the group consisting of renal disease, macular degeneration, degenerative joint disease, traumatic brain or spinal cord injury, stroke, atherosclerosis, arthritis, emphysema, osteoporosis, cardiomyopathy, cirrhosis, degenerative nerve disease, Holt-Oram disease, eye disease, diabetic nephropathy, degenerative bone disease, liver disease, periodontal disease, diabetes, cardiovascular disease, inflammatory disease, immune disease, skeletal disease, reproductive disease, haematopoietic disease, cellular damage due to ionizing radiation, cellular damage due to hypoxia and cancer. According to one embodiment, the tissue regeneration is selected from the group consisting of muscle, dendritic tissue, nerve, kidney, brain, bone, skin, lung, muscle, ovary, testes, heart, spleen, cartilage, nerve, peridontal, dentin, liver, vascular, connective, lymphatic, haematopoietic, and renal tissue.

According to another aspect, the disclosure features a method of treating or preventing a disease or disorder associated with fibrosis in a subject, the method comprising administering to a subject in need of treatment at least one TDFRP in combination with an additional agent, wherein the additional agent is selected from the group consisting of: an inhibitor of angiotensin converting enzyme (ACE), a neprilysin inhibitor and an angiotensin receptor-neprilysin inhibitor, wherein the TDFRP and additional agent are administered in an amount effective to treat the fibrosis in the subject. According to one embodiment, the fibrosis is selected from the group consisting of pulmonary fibrosis, renal fibrosis and hepatic fibrosis. According to another embodiment, the pulmonary fibrosis is idiopathic pulmonary fibrosis. According to one embodiment, the fibrosis is associated with a disease or condition selected from the group consisting of atherosclerosis, cardiac failure, cardiac arrhythmia, myocardial infarction, peripheral vascular disease, diabetes, chronic renal disease, pulmonary fibrosis, liver failure, and Alzheimer's disease. According to another embodiment, the disease or condition is a chronic disease or condition. According to one embodiment, treating the pulmonary fibrosis comprises restoring the function of the pulmonary tissue. According to one embodiment, treating the renal fibrosis comprises restoring the function of the renal tissue. According to one embodiment, treating the hepatic fibrosis comprises restoring the function of the hepatic tissue. According to one embodiment of the embodiments and aspects herein, the TDFRP is selected from the group consisting of: SEQ ID NO: 1, SEQ ID NO: 2 and SEQ ID NO: 3. According to one embodiment of the embodiments and aspects herein, the TDFRP is a Multiple Domain TDFRP. According to one embodiment of the embodiments and aspects herein, the angiotensin converting enzyme (ACE) inhibitor is selected from the group consisting of captopril, zofenopril, enalapril, ramipril, quinapril, perindopril, lisinopril, benazepril, imidapril, trandolapril, fosinopril, moexipril, cilazapril, spirapril, temocapril, alacepril, ceronapril, delepril, moveltipril, and combinations thereof. According to one embodiment of the embodiments and aspects herein, the neprilysin inhibitor is selected from the group consisting of thiorphan, candoxatril, and candoxatrilat. According to one embodiment of the embodiments and aspects herein, the angiotensin receptor-neprilysin inhibitor is sacubitril/valsartan. According to one embodiment of the embodiments and aspects herein, the TDFRP and the additional agent are administered separately, sequentially and/or concurrently. According to one embodiment of the embodiments and aspects herein, the TDFRP is provided in the same composition as the additional agent. According to one embodiment of the embodiments and aspects herein, the TDFRP is provided in a separate composition as the additional agent. According to one embodiment of the embodiments and aspects herein, the TDFRP is administered by intravenous, intraperitoneal, intramuscular, subcutaneous, topical, inhalation or other routes. According to one embodiment of the embodiments and aspects herein, the additional agent is administered by intravenous, intraperitoneal, intramuscular, subcutaneous, topical, inhalation or other routes. According to one embodiment of the embodiments and aspects herein, the method further comprises the administration of a therapeutic agent selected from the group consisting of anti-neoplastic agents, antibiotics, vaccines, immunosuppressive agents, antihypertensive agents and mediators of the hedgehog signaling pathway.

According to another aspect, the disclosure features a method of increasing the serum half-life of a tissue differentiation factor related polypeptide (TDFRP) in a subject, the method comprising administering to the subject at least one tissue differentiation factor related polypeptide (TDFRP) in combination with an additional agent, wherein the additional agent is selected from the group consisting of an inhibitor of angiotensin converting enzyme (ACE), a neprilysin inhibitor and an angiotensin receptor-neprilysin inhibitor, wherein administration of the TDFRP and additional agent increases the serum half-life of the TDFRP compared to administration of the TDFRP alone. According to one embodiment, the serum half-life is increased 2-fold or more when the TDFRP is administered with the additional agent. According to one embodiment, the serum half-life is increased 5-fold or more when the TDFRP is administered with the additional agent. According to one embodiment, the serum half-life is increased 10-fold or more when the TDFRP is administered with the additional agent. According to one embodiment, the subject has a tissue differentiation factor-associated disorder or disease. According to one embodiment, the subject has a disease or disorder associated with fibrosis.

According to another aspect, the present disclosure features a composition comprising at least one TDFRP and an additional agent, wherein the additional agent is selected from the group consisting of an inhibitor of angiotensin converting enzyme (ACE), a neprilysin inhibitor and an angiotensin receptor-neprilysin inhibitor. According to one embodiment, the composition is a pharmaceutical composition comprising the composition comprising at least one TDFRP and an additional agent, wherein the additional agent is selected from the group consisting of an inhibitor of angiotensin converting enzyme (ACE), a neprilysin inhibitor and an angiotensin receptor-neprilysin inhibitor and a pharmaceutical excipient.

According to another aspect, the disclosure features a kit comprising ta pharmaceutical composition comprising the composition comprising at least one TDFRP and an additional agent, wherein the additional agent is selected from the group consisting of an inhibitor of angiotensin converting enzyme (ACE), a neprilysin inhibitor and an angiotensin receptor-neprilysin inhibitor and a pharmaceutical excipient and instructions for use in treating or preventing a tissue differentiation factor-associated disorder or disease.

According to another aspect, the disclosure features a kit comprising ta pharmaceutical composition comprising the composition comprising at least one TDFRP and an additional agent, wherein the additional agent is selected from the group consisting of an inhibitor of angiotensin converting enzyme (ACE), a neprilysin inhibitor and an angiotensin receptor-neprilysin inhibitor and a pharmaceutical excipient and instructions for use in treating or preventing fibrosis in a subject.

### BRIEF DESCRIPTION OF THE DRAWINGS

FIG. 1 is a graph that shows the human plasma stability over time of tissue differentiation factor related polypeptide (TDFRP) THR-184 alone or in combination with Enalaprilat.
FIG. 2 is a graph that shows the percent of TDFRP polypeptide THR-184 (1000 ng/ml) remaining in human plasma after incubation with Lisinopril or Enalaprilat for 10 minutes at 37°C. Concentration of Enalaprilat is shown on the x-axis (mg/ml).
FIG. 3 is a graph that shows the percent of TDFRP polypeptides THR-184.TFA (trifluoroacetic acid) and THR-204.AC (acetic acid) (1000 ng/ml) remaining in human plasma after incubation with Enalaprilat for 60 minutes at 37°C. Concentration of Enalaprilat is shown on the x-axis (mg/ml).

### DETAILED DESCRIPTION

The present disclosure is based, in part, on the finding that tissue differentiation factor related polypeptides (TDFRPs), and analogs, homologs or variants thereof, which have been shown to have a short serum half-life, with large variability in AUC and peak serum concentrations in humans, when combined with additional agents (e.g. angiotensin-converting enzyme (ACE) inhibitors), have an extended serum half-life. According to certain embodiments, the serum half-life of the TDFRPs, when combined with an additional agent (e.g. inhibitors of angiotensin converting enzyme (ACE), neprilysin inhibitors or angiotensin receptor-neprilysin inhibitors), can be extended, in certain embodiments, by at least 2- to at least 10- fold. Thus, the combination of TDFRPs with an additional agent extends the exposure of the receptors to the TDFRPs, which allows the TDFRPs to be more effective as a therapeutic.

### Definitions

As used in this specification and the appended claims, the singular forms "a", "an" and "the" include plural references unless the content clearly dictates otherwise.

The use of the alternative (e.g., "or") should be understood to mean either one, both, or any combination thereof of the alternatives.

As used herein, the term "about," when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

As used herein, any concentration range, percentage range, ratio range, or integer range is to be understood to include the value of any integer within the recited range and, when appropriate, fractions thereof (such as one tenth and one hundredth of an integer), unless otherwise indicated.

As used herein, "comprise," "comprising," and "comprises" and "comprised of" are meant to be synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

As used herein, the terms "such as", "for example" and the like are intended to refer to exemplary embodiments and not to limit the scope of the present disclosure.

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, preferred materials and methods are described herein.

As used herein, "administration," "administering" and variants thereof refers to introducing a composition or agent into a subject and includes concurrent and sequential introduction of a composition or agent. "Administration" can refer, e.g., to therapeutic, pharmacokinetic, diagnostic, research, placebo, and experimental methods. "Administration" also encompasses in vitro and ex vivo treatments. The introduction of a composition or agent into a subject is by any suitable route, including orally, pulmonarily, intranasally, parenterally (intravenously, intramuscularly, intraperitoneally, or subcutaneously), rectally, intralymphatically, or topically. Administration includes self-administration and the administration by another. Administration can be carried out by any suitable route. A suitable route of administration allows the composition or the agent to perform its intended function. For example, if a suitable route is intravenous, the composition is administered by introducing the composition or agent into a vein of the subject.

As used herein, the term "analog" is meant to refer to a composition that differs from the compound of the present disclosure but retains essential properties thereof. A non-limiting example of this is a polypeptide or peptide or peptide fragment that includes non-natural amino acids, peptidomimetics, unusual amino acids, amide bond isosteres.

As used herein, the term "cancer" refers to diseases in which abnormal cells divide without control and are able to invade other tissues. There are more than 100 different types of cancer. Most cancers are named for the organ or type of cell in which they start - for example, cancer that begins in the colon is called colon cancer; cancer that begins in melanocytes of the skin is called melanoma. Cancer types can be grouped into broader categories. The main categories of cancer include: carcinoma (meaning a cancer that begins in the skin or in tissues that line or cover internal organs, and its subtypes, including adenocarcinoma, basal cell carcinoma, squamous cell carcinoma, and transitional cell carcinoma); sarcoma (meaning a cancer that begins in bone, cartilage, fat, muscle, blood vessels, or other connective or supportive tissue); leukemia (meaning a cancer that starts in blood-forming tissue (e.g., bone marrow) and causes large numbers of abnormal blood cells to be produced and enter the blood; lymphoma and myeloma (meaning cancers that begin in the cells of the immune system); and central nervous system (CNS) cancers (meaning cancers that begin in the tissues of the brain and spinal cord). In certain embodiments, the cancer is selected from cancers including, but not limited to, ACUTE lymphoblastic leukemia (ALL), ACUTE myeloid leukemia (AML), anal cancer, bile duct cancer, bladder cancer, bone cancer, bowel cancer, brain tumour, breast cancer, cancer of unknown primary, cancer spread to bone, cancer spread to brain, cancer spread to liver, cancer spread to lung, carcinoid, cervical cancer, choriocarcinoma, chronic lymphocytic leukemia (CLL), chronic myeloid leukemia (CML), colon cancer, colorectal cancer, endometrial cancer, eye cancer, gallbladder cancer, gastric cancer, gestational trophoblastic tumour (GTT), hairy cell leukemia, head and neck cancer, Hodgkin lymphoma, kidney cancer, laryngeal cancer, leukemia, liver cancer, lung cancer, lymphoma, melanoma skin cancer, mesothelioma, men's cancer, molar pregnancy, mouth and oropharyngeal cancer, myeloma, nasal and sinus cancers, nasopharyngeal cancer, non hodgkin lymphoma (NHL), oesophageal cancer, ovarian cancer, pancreatic cancer, penile cancer, prostate cancer, rare cancers, rectal cancer, salivary gland cancer, secondary cancers, skin cancer (non melanoma), soft tissue sarcoma, stomach cancer, testicular cancer, thyroid cancer, unknown primary cancer, uterine cancer, vaginal cancer, and vulval cancer. According to one embodiment, the cancer is prostate cancer. According to one embodiment, the cancer is breast cancer.

As used herein, an "effective amount" of a compound is meant to refer to a quantity sufficient to achieve a desired therapeutic and/or prophylactic effect, for example, an amount which results in the prevention of or a decrease in the symptoms associated with a disease that is being treated, *e.g.,* the diseases associated with TGF-beta superfamily polypeptides described herein. The amount of compound administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. Typically, an effective amount of the compounds of the present disclosure, sufficient for achieving a therapeutic or prophylactic effect, range from about 0.000001 mg per kilogram body weight per day, to about 10,000 mg per kilogram body weight per day. Preferably, the dosage ranges are from about 0.0001 mg per kilogram body weight per day to about 100 mg per kilogram body weight per day. The compounds of the present disclosure can also be administered in combination with each other, or with one or more additional therapeutic compounds.

As used herein, the terms "ischemia" or "ischemic episode," are meant to refer to any circumstance that results in a deficient supply of blood or oxygen to a tissue. Thus, a central nervous system ischemic episode results from an insufficiency or interruption in the blood or oxygen supply to any locus of the brain such as, but not limited to, a locus of the cerebrum, cerebellum or brain stem. The spinal cord, which is also a part of the central nervous system, is equally susceptible to ischemia resulting from diminished blood flow or lack of oxygen. An ischemic episode may be caused by a constriction or obstruction of a blood vessel, as occurs in the case of a thrombus or embolus. Alternatively, the ischemic episode may result from any form of compromised cardiac function, including cardiac arrest, as described above. Where the deficiency is sufficiently severe and prolonged, it can lead to disruption of physiologic function, subsequent death of neurons, and necrosis (infarction) of the affected areas. The extent and type of neurologic abnormality resulting from the injury depend on the location and size of the infarct or the focus of ischemia. Where the ischemia is associated with a stroke, it can be either global or focal in extent. Ischemia can occur in other tissues or organs including kidney. Restoration of blood flow or reperfusion leads to a series of cellular responses that are known to cause tissue damage.

As used herein, an "isolated" or "purified" polypeptide or polypeptide or biologically-active portion thereof is substantially free of cellular material or other contaminating polypeptides from the cell or tissue source from which the tissue differentiation factor-related polypeptide is derived, or substantially free from chemical precursors or other chemicals when chemically synthesized.

As used herein, the terms "polypeptide", "peptide" and "protein" are used interchangeably herein to refer to a natural or synthetic peptide containing two or more amino acids linked typically via the carboxy group of one amino acid and the amino group of another amino acid. As will be appreciated by those having skill in the art, the above definition is not absolute and polypeptides or peptides can include other examples where one or more amide bonds could be replaced by other bonds, for example, isosteric amide bonds. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" also are inclusive of modifications including, but not limited to, glycosylation, lipid attachment, sulfation, gamma-carboxylation of glutamic acid residues, hydroxylation, and ADP-ribosylation. It will be appreciated, as is well known and as noted above, that polypeptides may not be entirely linear. For instance, polypeptides may be branched as a result of ubiquitination, and they may be circular, with or without branching, generally as a result of posttranslational events, including natural processing event and events brought about by human manipulation which do not occur naturally. Circular, branched and branched circular polypeptides may be synthesized by non-translation natural process and by entirely synthetic methods, as well.

As used herein, the term "pharmaceutically acceptable carrier" includes any of the standard pharmaceutical carriers, such as a phosphate buffered saline solution, water, emulsions such as an oil/water or water/oil, and various types of wetting agents. The term also encompasses any of the agents approved by a regulatory agency of the US Federal government or listed in the US Pharmacopeia for use in animals, including humans, as well as any carrier or diluent that does not cause significant irritation to a subject and does not abrogate the biological activity and properties of the administered compound.

As used herein, the term " small molecule" is meant to refer to a composition that has a molecular weight of less than about 5 kDa and more preferably less than about 2 kDa. Small molecules can be, *e.g.,* nucleic acids, peptides, polypeptides, glycopeptides, peptidomimetics, carbohydrates, lipids, lipopolysaccharides, combinations of these, or other organic or inorganic molecules.

As used herein, the terms "subject," "individual," "host," and "patient," are used interchangeably herein and refer to any mammalian subject for whom diagnosis, treatment, or therapy is desired, particularly humans. The methods described herein are applicable to both human therapy and veterinary applications. According to some embodiments, the subject is a mammal, and in other embodiments the subject is a human. A "subject in need" is meant to refer to a subject that (i) will be administered a TDFRP and additional agent *(e.g.* an ACE inhibitor) according to the disclosure, (ii) is receiving a TDFRP and additional agent *(e.g.* an ACE inhibitor) according to the disclosure; or (iii) has received an aa TDFRP and additional agent *(e.g.* an ACE inhibitor) according to the disclosure, unless the context and usage of the phrase indicates otherwise.

As used herein, the terms "therapeutic amount", "therapeutically effective amount", an "amount effective", or "pharmaceutically effective amount" of an active agent (*e.g.* a TDFRP and additional agent *(e.g.* an ACE inhibitor), as described herein, are used interchangeably to refer to an amount that is sufficient to provide the intended benefit of treatment. However, dosage levels are based on a variety of factors, including the type of injury, the age, weight, sex, medical condition of the patient, the severity of the condition, the route of administration, and the particular active agent employed. Thus the dosage regimen may vary widely, but can be determined routinely by a physician using standard methods. Additionally, the terms "therapeutic amount", "therapeutically effective amounts" and "pharmaceutically effective amounts" include prophylactic or preventative amounts of the compositions of the disclosure. In prophylactic or preventative applications of the disclosure, pharmaceutical compositions or medicaments are administered to a patient susceptible to, or otherwise at risk of, a disease, disorder or condition in an amount sufficient to eliminate or reduce the risk, lessen the severity, or delay the onset of the disease, disorder or condition, including biochemical, histologic and/or behavioral symptoms of the disease, disorder or condition, its complications, and intermediate pathological phenotypes presenting during development of the disease, disorder or condition. It is generally preferred that a maximum dose be used, that is, the highest safe dose according to some medical judgment. The terms "dose" and "dosage" are used interchangeably herein.

As used herein, the term "Transforming Growth Factor- beta (TGF-β) superfamily of polypeptides," is meant to refer to a superfamily of polypeptide factors with pleiotropic functions that is composed of many multifunctional cytokines which includes, but is not limited to, TGF-βs, activins, inhibins, anti-müllerian hormone (AMH), mullerian inhibiting substance (MIS), bone morphogenetic proteins (BMPs), and myostatin. The highly similar TGF-β isoforms TGF-β1, TGF-β2, and TGF-β3 potently inhibit cellular proliferation of many cell types, including those from epithelial origin. Most mesenchymal cells, however, are stimulated in their growth by TGF-β. In addition, TGF-βs strongly induce extracellular matrix synthesis and integrin expression, and modulate immune responses. BMPs, also known as osteogenic proteins (OPs), are potent inducers of bone and cartilage formation and play important developmental roles in the induction of ventral mesoderm, differentiation of neural tissue, and organogenesis. Activins, named after their initial identification as activators of follicle-stimulating hormone (FSH) secretion from pituitary glands, are also known to promote erythropoiesis, mediate dorsal mesoderm induction, and contribute to survival of nerve cells. Several growth factors belonging to the TGF-β superfamily play important roles in embryonic patterning and tissue homeostasis. Their inappropriate functioning has been implicated in several pathological situations like fibrosis, rheumatoid arthritis, and carcinogenesis. The term, tissue differentiation factor (TDF), as used herein, includes, but is not limited to, all members of the TGF-beta superfamily of polypeptides. TGF-beta superfamily polypeptides can be antagonists or agonists of TGF-beta superfamily receptors.

As used herein, the term "Transforming Growth Factor- beta (TGF-beta) superfamily receptors," is meant to refer to polypeptide receptors that mediate the pleiotropic effects of transforming growth factor-β (TGF-β) superfamily polypeptides, as well as fragments, analogs and homologs thereof. Such receptors may include, but are not limited to, distinct combinations of Type I and Type II serine/threonine kinase receptors. The term, tissue differentiation factor receptor (TDF), as used herein, includes, but is not limited to, all members of the TGF-beta superfamily of receptors.

As used herein, the terms "treat," "treating," and/or "treatment" include abrogating, substantially inhibiting, slowing or reversing the progression of a condition, substantially ameliorating clinical symptoms of a condition, or substantially preventing the appearance of clinical symptoms of a condition, obtaining beneficial or desired clinical results. Treating further refers to accomplishing one or more of the following: (a) reducing the severity of the disorder; (b) limiting development of symptoms characteristic of the disorder(s) being treated; (c) limiting worsening of symptoms characteristic of the disorder(s) being treated; (d) limiting recurrence of the disorder(s) in patients that have previously had the disorder(s); and (e) limiting recurrence of symptoms in patients that were previously asymptomatic for the disorder(s).

Beneficial or desired clinical results, such as pharmacologic and/or physiologic effects include, but are not limited to, preventing the disease, disorder or condition from occurring in a subject that may be predisposed to the disease, disorder or condition but does not yet experience or exhibit symptoms of the disease (prophylactic treatment), alleviation of symptoms of the disease, disorder or condition, diminishment of extent of the disease, disorder or condition, stabilization (*i.e*., not worsening) of the disease, disorder or condition, preventing spread of the disease, disorder or condition, delaying or slowing of the disease, disorder or condition progression, amelioration or palliation of the disease, disorder or condition, and combinations thereof, as well as prolonging survival as compared to expected survival if not receiving treatment.

As used herein the term "therapeutic effect" refers to a consequence of treatment, the results of which are judged to be desirable and beneficial. A therapeutic effect can include, directly or indirectly, the arrest, reduction, or elimination of a disease manifestation. A therapeutic effect can also include, directly or indirectly, the arrest reduction or elimination of the progression of a disease manifestation.

For any therapeutic agent described herein the therapeutically effective amount may be initially determined from preliminary in vitro studies and/or animal models. A therapeutically effective dose may also be determined from human data. The applied dose may be adjusted based on the relative bioavailability and potency of the administered compound. Adjusting the dose to achieve maximal efficacy based on the methods described above and other well-known methods is within the capabilities of the ordinarily skilled artisan. General principles for determining therapeutic effectiveness, which may be found in Chapter 1 of Goodman and Gilman's The Pharmacological Basis of Therapeutics, 10th Edition, McGraw-Hill (New York) (2001), incorporated herein by reference, are summarized below.

Pharmacokinetic principles provide a basis for modifying a dosage regimen to obtain a desired degree of therapeutic efficacy with a minimum of unacceptable adverse effects. In situations where the drug's plasma concentration can be measured and related to the therapeutic window, additional guidance for dosage modification can be obtained. Drug products are considered to be pharmaceutical equivalents if they contain the same active ingredients and are identical in strength or concentration, dosage form, and route of administration. Two pharmaceutically equivalent drug products are considered to be bioequivalent when the rates and extents of bioavailability of the active ingredient in the two products are not significantly different under suitable test conditions.

As used herein, the term "variant," is meant to refer to a compound that differs from the compound of the present disclosure, but retains essential properties thereof. A non-limiting example of this is a polynucleotide or polypeptide compound having conservative substitutions with respect to the reference compound, commonly known as degenerate variants. Another non-limiting example of a variant is a compound that is structurally different, but retains the same active domain of the compounds of the present disclosure. Variants include N-terminal or C-terminal extensions, capped amino acids, modifications of reactive amino acid side chain functional groups, *e.g*., branching from lysine residues, pegylation, and/or truncations of a polypeptide compound. Generally, variants are overall closely similar, and in many regions, identical to the compounds of the present disclosure. Accordingly, the variants may contain alterations in the coding regions, non-coding regions, or both.

### COMPOSITIONS

According to one aspect, the present disclosure provides compositions comprising at least one TDFRP and an additional agent. According to some embodiments, the additional agent is an angiotensin converting enzyme (ACE). According to some embodiments, the additional agent is a neprilysin inhibitor. According to some embodiments, the additional agent is an angiotensin receptor-neprilysin inhibitor. The present disclosure provides in another aspect treating a subject with a composition comprising at least one TDFRP and an additional agent. According to some embodiments, the TDFRP is provided in the same composition as the additional agent. According to other embodiments, the TDFRP is provided in a separate composition as the additional agent. According to some embodiments, the additional agent can be an angiotensin converting enzyme (ACE). According to some embodiments, the additional agent Isa neprilysin inhibitor. According to some embodiments, the additional agent is an angiotensin receptor-neprilysin inhibitor.

### Tissue Differentiation Factor Related Polypeptides (TDFRP (TDFRPs)

The present disclosure provides compounds that are functional analogs of tissue differentiation factors, *i.e.,* compounds that functionally mimic TGF-beta superfamily proteins, for example by acting as TGF-beta superfamily receptor agonists, and preferentially bind to select ALK receptor(s). The present compounds are called TDFRPs, and include small molecules, more particularly polypeptides.

According to one embodiment, the TDFRP compound has the general structure identified as SEQ ID NOs: 1-208, disclosed in International Publication No. WO/2003/106656, incorporated by reference in its entirety herein. According to one embodiment, a TDFRP compound includes an analog or homolog of SEQ ID NOs: 1-208. Compounds of the present disclosure include those with homology to SEQ ID Nos: 1-208, for example, preferably 50% or greater amino acid identity, more preferably 75% or greater amino acid identity, and even more preferably 90% or greater amino acid identity. The compounds of the present disclosure also include one or more polynucleotides encoding SEQ ID Nos:1-208, including degenerate variants thereof. Accordingly, nucleic acid sequences capable of hybridizing at low stringency with any nucleic acid sequences encoding SEQ ID Nos:1-208 are considered to be within the scope of the disclosure.

According to one embodiment, the TDFRP compound has the general structure identified as SEQ ID NOs:1-347, disclosed in International Publication No. WO/2007/035872, incorporated by reference in its entirety herein. According to one embodiment, a TDFRP compound includes an analog or homolog of SEQ ID NOs:1-347. Compounds of the present disclosure include those with homology to SEQ ID Nos:1-347, for example, preferably 50% or greater amino acid identity, more preferably 75% or greater amino acid identity, and even more preferably 90% or greater amino acid identity. The compounds of the present disclosure also include one or more polynucleotides encoding SEQ ID Nos:1-347, including degenerate variants thereof. Accordingly, nucleic acid sequences capable of hybridizing at low stringency with any nucleic acid sequences encoding SEQ ID Nos:1-347 are considered to be within the scope of the disclosure.

According to one embodiment, the TDFRP compound has the general structure identified as SEQ ID NOs:1-314, disclosed in International Publication No. WO/2006/009836, incorporated by reference in its entirety herein. According to one embodiment, a TDFRP compound includes an analog or homolog of SEQ ID NOs:1-314. Compounds of the present disclosure include those with homology to SEQ ID Nos:1-314, for example, preferably 50% or greater amino acid identity, more preferably 75% or greater amino acid identity, and even more preferably 90% or greater amino acid identity. The compounds of the present disclosure also include one or more polynucleotides encoding SEQ ID Nos:1-314, including degenerate variants thereof. Accordingly, nucleic acid sequences capable of hybridizing at low stringency with any nucleic acid sequences encoding SEQ ID Nos:1-314 are considered to be within the scope of the disclosure.

Sequence identity can be measured using sequence analysis software (Sequence Analysis Software Package of the Genetics Computer Group, University of Wisconsin Biotechnology Center, 1710 University Avenue, Madison, Wis. 53705), with the default parameters therein.

In the case of polypeptide sequences, which are less than 100% identical to a reference sequence, the non-identical positions are preferably, but not necessarily, conservative substitutions for the reference sequence. Conservative substitutions typically include substitutions within the following groups: glycine and alanine; valine, isoleucine, and leucine; aspartic acid and glutamic acid; asparagine and glutamine; serine and threonine; lysine and arginine; and phenylalanine and tyrosine. Thus, included in the disclosure are peptides having mutated sequences such that they remain homologous, *e.g.,* in sequence, in structure, in function, and in antigenic character or other function, with a polypeptide having the corresponding parent sequence. Such mutations can, for example, be mutations involving conservative amino acid changes, *e.g.,* changes between amino acids of broadly similar molecular properties. For example, interchanges within the aliphatic group alanine, valine, leucine and isoleucine can be considered as conservative. Sometimes substitution of glycine for one of these can also be considered conservative. Other conservative interchanges include those within the aliphatic group aspartate and glutamate; within the amide group asparagine and glutamine; within the hydroxyl group serine and threonine; within the aromatic group phenylalanine, tyrosine and tryptophan; within the basic group lysine, arginine and histidine; and within the sulfur-containing group methionine and cysteine. Sometimes substitution within the group methionine and leucine can also be considered conservative. Preferred conservative substitution groups are aspartate-glutamate; asparagine-glutamine; valine-leucine-isoleucine; alanine-valine; phenylalanine- tyrosine; and lysine-arginine.

The disclosure also provides for compounds having altered sequences including insertions such that the overall amino acid sequence is lengthened, while the compound still retains the appropriate TDF agonist or antagonist properties. Additionally, altered sequences may include random or designed internal deletions that truncate the overall amino acid sequence of the compound, however the compound still retains its TDF-like functional properties. According to certain embodiments, one or more amino acid residues within the sequence are replaced with other amino acid residues having physical and/or chemical properties similar to the residues they are replacing. Preferably, conservative amino acid substitutions are those wherein an amino acid is replaced with another amino acid encompassed within the same designated class, as will be described more thoroughly below. Insertions, deletions, and substitutions are appropriate where they do not abrogate the functional properties of the compound. Functionality of the altered compound can be assayed according to the *in vitro* and *in vivo* assays described below that are designed to assess the TDF-like properties of the altered compound.

According to some embodiments, particularly preferred peptides include but are not limited to, the following:

| **Sequence** | **THR No.** | **SEQ ID NO:** |
|---|---|---|
| CYFDDSSNVLCKKYRS (123) | 123 | 1 |
| CYFDDSSQVICKKYRS | 204 | 2 |
| CYYDNSSSVLCKRYRS | 184 | 3 |

In yet another embodiment, the peptide of the invention can be: CYYDNSSSVLCKR**X₁₄**RS (SEQ ID NO:4), wherein **X₁₄** is D-Tyr.

### TDFRP Recombinant Expression Vectors

According to one aspect, the disclosure includes vectors containing one or more nucleic acid sequences encoding a TDFRP compound. For recombinant expression of one or more the polypeptides of the disclosure, the nucleic acid containing all or a portion of the nucleotide sequence encoding the polypeptide is inserted into an appropriate cloning vector, or an expression vector i*(i.e.,* a vector that contains the necessary elements for the transcription and translation of the inserted polypeptide coding sequence) by recombinant DNA techniques well known in the art and as detailed below.

In general, expression vectors useful in recombinant DNA techniques are often in the form of plasmids. In the present specification, "plasmid" and "vector" can be used interchangeably as the plasmid is the most commonly used form of vector. However, the disclosure is intended to include such other forms of expression vectors that are not technically plasmids, such as viral vectors (*e.g.,* replication defective retroviruses, adenoviruses and adeno-associated viruses), which serve equivalent functions. Such viral vectors permit infection of a subject and expression in that subject of a compound.

The recombinant expression vectors of the disclosure comprise a nucleic acid encoding a compound with TDF-like properties in a form suitable for expression of the nucleic acid in a host cell, which means that the recombinant expression vectors include one or more regulatory sequences, selected on the basis of the host cells to be used for expression that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g.,* in an *in vitro* transcription/translation system or in a host cell when the vector is introduced into the host cell).

The term "regulatory sequence" is intended to include promoters, enhancers and other expression control elements (*e.g.,* polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g.,* tissue-specific regulatory sequences). It will be appreciated by those skilled in the art that the design of the expression vector can depend on such factors as the choice of the host cell to be transformed, the level of expression of polypeptide desired, *etc.* The expression vectors of the disclosure can be introduced into host cells to thereby produce polypeptides or peptides, including fusion polypeptides, encoded by nucleic acids as described herein (*e.g.,* TDFRP compounds and TDFRP-derived fusion polypeptides, *etc*.).

### TDFRP-Expressing Host Cells

According to another aspect, the present disclosure pertains to TDFRP-expressing host cells, which contain a nucleic acid encoding one or more TDFRP compounds. The recombinant expression vectors of the disclosure can be designed for expression of TDFRP compounds in prokaryotic or eukaryotic cells. For example, TDFRP compounds can be expressed in bacterial cells such as *Escherichia coli,* insect cells (using baculovirus expression vectors), fungal cells, *e.g.,* yeast, yeast cells or mammalian cells. Suitable host cells are discussed further in Goeddel, GENE EXPRESSION TECHNOLOGY: METHODS IN ENZYMOLOGY 185, Academic Press, San Diego, CA. (1990). Alternatively, the recombinant expression vector can be transcribed and translated *in vitro,* for example using T7 promoter regulatory sequences and T7 polymerase.

Expression of polypeptides in prokaryotes is most often carried out in *Escherichia coli* with vectors containing constitutive or inducible promoters directing the expression of either fusion or non-fusion polypeptides. Fusion vectors add a number of amino acids to a polypeptide encoded therein, usually to the amino terminus of the recombinant polypeptide. Such fusion vectors typically serve three purposes: (i) to increase expression of recombinant polypeptide; (ii) to increase the solubility of the recombinant polypeptide; and (iii) to aid in the purification of the recombinant polypeptide by acting as a ligand in affinity purification. Often, in fusion expression vectors, a proteolytic cleavage site is introduced at the junction of the fusion moiety and the recombinant polypeptide to enable separation of the recombinant polypeptide from the fusion moiety subsequent to purification of the fusion polypeptide. Such enzymes, and their cognate recognition sequences, include Factor Xa, thrombin and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding polypeptide, or polypeptide A, respectively, to the target recombinant polypeptide.

Examples of suitable inducible non-fusion *E. coli* expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

One strategy to maximize recombinant polypeptide expression in *E. coli* is to express the polypeptide in host bacteria with an impaired capacity to proteolytically cleave the recombinant polypeptide. *See, e.g.,* Gottesman, GENE EXPRESSION TECHNOLOGY: METHODS IN ENZYMOLOGY 185, Academic Press, San Diego, CA. (1990) 119-128. Another strategy is to alter the nucleic acid sequence of the nucleic acid to be inserted into an expression vector so that the individual codons for each amino acid are those preferentially utilized in the expression host, *e.g., E. coli* (*see, e.g.,* Wada, et al., 1992. Nucl. Acids Res. 20: 2111-2118). Such alteration of nucleic acid sequences of the disclosure can be carried out by standard DNA synthesis techniques.

In another embodiment, the TDFRP expression vector is a yeast expression vector. Examples of vectors for expression in yeast *Saccharomyces cerivisae* include pYepSec1 (Baldari, et al., 1987. EMBO J. 6: 229-234), pMFa (Kurjan and Herskowitz, 1982. Cell 30: 933-943), pJRY88 (Schultz et al., 1987. Gene 54: 113-123), pYES2 (Invitrogen Corporation, San Diego, CA.), and picZ (Invitrogen Corp, San Diego, CA.). Alternatively, TDFRP can be expressed in insect cells using baculovirus expression vectors. Baculovirus vectors available for expression of polypeptides in cultured insect cells (*e.g.,* SF9 cells) include the pAc series (Smith, et al., 1983. Mol. Cell. Biol. 3: 2156-2165) and the pVL series (Lucklow and Summers, 1989. Virology 170: 31-39).

In yet another embodiment, a nucleic acid of the disclosure is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells *see, e.g.,* Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, NY, 1989.

In another embodiment, the recombinant mammalian expression vector is capable of directing expression of the nucleic acid preferentially in a particular cell type (*e.g.,* tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Banerji, et al., 1983. Cell 33: 729-740; Queen and Baltimore, 1983. Cell 33: 741-748), neuron-specific promoters (*e.g.,* the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (*e.g.,* milk whey promoter; U.S. Pat. No. 4,873,316 and European Application Publication No. 264,166). Developmentally-regulated promoters are also encompassed, *e.g.,* the murine hox promoters (Kessel and Gruss, 1990. Science 249: 374-379) and the α-fetoprotein promoter (Campes and Tilghman, 1989. Genes Dev. 3: 537-546).

The disclosure further provides a recombinant expression vector comprising a DNA molecule of the disclosure cloned into the expression vector in an antisense orientation. That is, the DNA molecule is operatively linked to a regulatory sequence in a manner that allows for expression (by transcription of the DNA molecule) of an RNA molecule that is antisense to a TDRFP mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, et al., "Antisense RNA as a molecular tool for genetic analysis," Reviews-Trends in Genetics, Vol. 1(1) 1986.

Another aspect of the disclosure pertains to host cells into which a recombinant expression vector of the disclosure has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, TDFRP can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid *(e.g.,* DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.,* resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding TDFRP or can be introduced on a separate vector. Cells stably transfected with the introduced nucleic acid can be identified by drug selection (*e.g.,* cells that have incorporated the selectable marker gene will survive, while the other cells die).

A host cell that includes a compound of the disclosure, such as a prokaryotic or eukaryotic host cell in culture can be used to produce (*i.e.,* express) recombinant TDFRP. According to one embodiment, the method comprises culturing the host cell of disclosure (into which a recombinant expression vector encoding TDFRP has been introduced) in a suitable medium such that TDFRP is produced. According to another embodiment, the method further comprises the step of isolating TDFRP from the medium or the host cell. Purification of recombinant polypeptides is well-known in the art and include ion-exchange purification techniques, or affinity purification techniques, for example with an antibody to the compound.

### TDFRP-derived Chimeric and Fusion Polypeptides

The present disclosure also provides for compounds that are TDFRP-derived chimeric or fusion polypeptides. As used herein, a TDFRP-derived "chimeric polypeptide" or "fusion polypeptide" comprises a TDFRP operatively-linked to a polypeptide having an amino acid sequence corresponding to a polypeptide that is not substantially homologous to the TDFRP, *e.g.,* a polypeptide that is different from the TDFRP and that is derived from the same or a different organism (*i.e.,* non-TDFRP). Within a TDFRP-derived fusion polypeptide, the TDFRP can correspond to all or a portion of a TDFRP. According to one embodiment, a TDFRP-derived fusion polypeptide comprises at least one biologically-active portion of a TDFRP, for example a fragment of SEQ ID Nos: 1-347. According to another embodiment, a TDFRP-derived fusion polypeptide comprises at least two biologically active portions of a TDFRP. In yet another embodiment, a TDFRP-derived fusion polypeptide comprises at least three biologically active portions of a TDFRP polypeptide. Within the fusion polypeptide, the term "operatively linked" is intended to indicate that the TDFRP polypeptide and the non-TDFRP polypeptide are fused in-frame with one another. The non-TDFRP polypeptide can be fused to the N-terminus or C-terminus of the TDFRP.

According to one embodiment, the fusion polypeptide is a GST-TDFRP fusion polypeptide in which the TDFRP sequences are fused to the N-or C-terminus of the GST (glutathione S-transferase) sequences. Such fusion polypeptides can facilitate the purification of recombinant TDFRP by affinity means.

According to another embodiment, the fusion polypeptide is a TDFRP polypeptide containing a heterologous signal sequence at its N-terminus. In certain host cells (*e.g.,* mammalian host cells), expression and/or secretion of TDFRP can be increased through use of a heterologous signal sequence.

According to yet another embodiment, the fusion polypeptide is a TDFRP-immunoglobulin fusion polypeptide in which the TDFRP sequences are fused to sequences derived from a member of the immunoglobulin superfamily. The TDFRP-immunoglobulin fusion polypeptides of the disclosure can be incorporated into pharmaceutical compositions and administered to a subject to inhibit an interaction between a TDF and a TDF receptor polypeptide on the surface of a cell, to thereby suppress TDF-mediated signal transduction *in vivo.* The TDFRP-immunoglobulin fusion polypeptides can be used to affect the bioavailability of a TDFRP, for example to target the compound to a particular cell or tissue having the requisite antigen. Inhibition of the TDF/TDF receptor interaction can be useful therapeutically for both the treatment of proliferative and differentiative disorders, as well as modulating (*e.g.,* promoting or inhibiting) cell survival.

### TDFRP1-linker-TDFRP2

The TDFRP compounds described herein contain multiple TDF-related polypeptides (*i.e.,* multiple domain TDF-related polypeptide compounds, hereinafter "TDFRP") with the general structure shown below:
TDFRP1-linker-TDFRP2
Where a first TDFRP domain (TDFRP1, *i.e.,* TDF-related polypeptide 1) is covalently linked via the C-terminus, N-terminus, or any position with a functionalizable side group, *e.g.,* lysine or aspartic acid to a linker molecule, which, in turn, is covalently linked to the N-terminus of a second TDFRP domain (TDFRP2).

The TDRFP domains are compounds that include small molecules. Variants, analogs, homologs, or fragments of these compounds, such as species homologs, are also included in the present disclosure, as well as degenerate forms thereof.

A first domain is linked to a second domain through a linker. The term "linker, " as used herein, refers to an element capable of providing appropriate spacing or structural rigidity, or structural orientation, alone, or in combination, to a first and a second domain, *e.g.,* TDFRP1 and TDFRP2, such that the biological activity of the TDFRP is preserved. For example, linkers may include, but are not limited to, a diamino alkane, a dicarboxylic acid, an amino carboxylic acid alkane, an amino acid sequence, *e.g.,* glycine polypeptide, a disulfide linkage, a helical or sheet-like structural element or an alkyl chain. According to one aspect the linker is not inert, *e.g.,* chemically or enzymatically cleavable *in vivo* or *in vitro.* In another aspect the linker is inert, *i.e.,* substantially unreactive *in vivo* or *in vitro, e.g.,* is not chemically or enzymatically degraded. Examples of inert groups which can serve as linking groups include aliphatic chains such as alkyl, alkenyl and alkynyl groups (*e.g.,* C1-C20), cycloalkyl rings (*e.g.,* C3-C10), aryl groups (carbocyclic aryl groups such as 1-naphthyl, 2-naphthyl, 1-anthracyl and 2-anthracyl and heteroaryl group such as N-imidazolyl, 2-imidazole, 2-thienyl, 3-thienyl, 2-furanyl, 3-furanyl, 2-pyridyl, 3-pyridyl, 4-pyridyl, 2-pyrimidy, 4-pyrimidyl, 2-pyranyl, 3-pyranyl, 3-pyrazolyl, 4-pyrazolyl, 5-pyrazolyl, 2-pyrazinyl, 2-thiazole, 4-thiazole, 5-thiazole, 2-oxazolyl, 4-oxazolyl, 5-oxazolyl, 2-benzothienyl, 3-benzothienyl, 2-benzofuranyl, 3-benzofuranyl, 2-indolyl, 3-indolyl, 2-quinolinyl, 3-quinolinyl, 2-benzothiazole, 2-benzooxazole, 2-benzimidazole, 2-quinolinyl, 3-quinolinyl, 1-isoquinolinyl, 3-quinolinyl, 1-isoindolyl, and 3-isoindolyl), non-aromatic heterocyclic groups (*e.g.,* 2-tetrahydrofuranyl, 3-tetrahydrofuranyl, 2-tetrahyrothiophenyl, 3-tetrahyrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl and 4-thiazolidinyl) and aliphatic groups in which one, two or three methylenes have been replaced with -O-, -S-, -NH-, -SO₂-, -SO- or -SO₂NH-.

The TDFRP compounds include small molecules, more particularly TDFRP compound domains, with the general structure identified herein, as detailed below. The TDFRP compound domains disclosed herein may be present in an TDFRP compound in any combination or orientation. Variants, analogs, homologs, or fragments of these TDFRP compound domains, such as species homologs, are also included in the present disclosure, as well as degenerate forms thereof. The TDFRP compound domains of the present disclosure may be capped on the N-terminus, or the C-terminus, or on both the N-terminus and the C-terminus. The TDFRP compounds may be pegylated, or modified, *e.g.,* branching, at any amino acid residue containing a reactive side chain, *e.g.,* lysine residue, or chemically reactive group on the linker. The TDFRP compound of the present disclosure may be linear or cyclized. The tail sequence of the TDFRP or TDFRP domains may vary in length. According to one aspect of the present disclosure, the TDFRP compounds of the disclosure are prodrugs, *i.e.,* the biological activity of the TDFRP compound is altered, *e.g.,* increased, upon contacting a biological system *in vivo* or *in vitro.*

The TDFRP compounds can contain natural amino acids, non-natural amino acids, d-amino acids and l-amino acids, and any combinations thereof. According to certain embodiments, the compounds of the disclosure can include commonly encountered amino acids, which are not genetically encoded. These non-genetically encoded amino acids include, but are not limited to, β-alanine (β-Ala) and other omega-amino acids such as 3-aminopropionic acid (Dap), 2,3-diaminopropionic acid (Dpr), 4-aminobutyric acid and so forth; α-aminoisobutyric acid (Aib); ε-aminohexanoic acid (Aha); δ-aminovaleric acid (Ava); N-methylglycine or sarcosine (MeGly); ornithine (Orn); citrulline (Cit); t-butylalanine (t-BuA); t-butylglycine (t-BuG); N-methylisoleucine (MeIle); phenylglycine (Phg); cyclohexylalanine (Cha); norleucine (Nle); 2-naphthylalanine (2-Nal); 4-chlorophenylalanine (Phe(4-Cl)); 2-fluorophenylalanine (Phe(2-F)); 3-fluorophenylalanine (Phe(3-F)); 4-fluorophenylalanine (Phe(4-F)); penicillamine (Pen); 1,2,3,4-tetrahydroisoquinoline-3-carboxylic acid (Tic); β-2-thienylalanine (Thi); methionine sulfoxide (MSO); homoarginine (hArg); N-acetyl lysine (AcLys); 2,3-diaminobutyric acid (Dab); 2,3-diaminobutyric acid (Dbu); p-aminophenylalanine (Phe(pNH₂)); N-methyl valine (MeVal); homocysteine (hCys) and homoserine (hSer). Non-naturally occurring variants of the compounds may be produced by mutagenesis techniques or by direct synthesis.

### Measurement of TDFRP Biological Activity

The biological activity, namely the agonist or antagonist properties of TDF polypeptides or TDFRP compounds can be characterized using any conventional *in vivo* and *in vitro* assays that have been developed to measure the biological activity of the TDFRP compound, a TDF polypeptide or a TDF signaling pathway component.
TGF-b/BMPs Superfamily members are associated with a number of cellular activities involved in injury responses and regeneration. TDFRP compounds can be used as agonists of BMPs or antagonists of TGF-b molecules to mediate activities that can prevent, repair or alleviate injurious responses in cells, tissues or organs. Key activities involved in mediating these effects would be anti-inflammatory, anti-apoptotic and anti-fibrotic properties. Several *in vitro* models for inflammation can be used to assess cytokine, chemokine and cell adhesion responses, which are well-documented markers of inflammation. Cellular injury induced by tumor necrosis factor-alpha (TNF-α)□ □, cisplatin, lipopolysaccharide (LPS) or other agents lead to the production of pro-inflammatory molecules (for example, IL-1, IL-6, IL-8, NF-kappaB) and adhesion molecules (for example, intercellular adhesion molecule-1 or ICAM-1). Furthermore, these agents induce chemokines (IL-6, IL-8, monocyte chemoattractant protein-1 or MCP-1 and RANTES), which cause immune cells to infiltrate tissues resulting in organ damage.

Apoptosis or programmed cell death is initiated through either a mitochondrial pathway, in response to stress factors or through a receptor-mediated pathway, triggered by the binding of ligands, such as TNF-α. Multiple factors contribute to the complex apoptotic process, including the infiltration neutrophils and other inflammatory cells that activate a class of enzymes known as caspases. Other useful markers of apoptosis are Bax and the human vascular anticoagulant, Annexin V, which binds to a protein that gets translocated from the inner to the outer plasma membrane in apoptotic cells.

In a typical experiment, the anti-apoptotic activity of TDFRP compounds can be assessed using *in vitro* models of apoptosis in cultured cells (for example, human kidney cells (HK-2) and heart muscle cells or cardiomyocytes). In kidney proximal tubule cells, a significant increase in the levels of Bax occurs in response to cell injury caused by cisplatin or other agents. In a typical experiment, the anti-apoptotic properties of TDFRP compounds can be demonstrated by the inhibition of Bax production induced by TNF-α or cisplatin. In addition, cisplatin treatment results in the expression of Annexin V in HK-2 cells.

Cardiomyocytes rarely proliferate in adult cardiac muscles and the loss of cardiac muscle cells can lead to permanent loss of cardiac function. Myocardial apoptosis, caused by injury to cardiomyoctes, contributes to or aggravates the development of myocardial dysfunction in various cardiac diseases. The chemotherapeutic agent, doxorubicin causes heart failure, a reduced number of functioning cardiac muscle cells, activation of caspase 3 and apoptosis. In a typical experiment, the anti-apoptotic activity of TDFRP compounds can be demonstrated by showing the inhibition of Bax and caspase-3 expression that was induced by doxorubicin, LPS or ischemia, as well as by showing an increase in the levels of phosphorylated Akt, a sensitive indicator of cardiomyocyte health.

In a typical experiment, the anti-fibrotic properties of TDFRP compounds can been demonstrated using *in vitro* and *in vivo* assays. For example, the reversal of tubular fibrosis induced by TGF-β in mouse kidney tubular epithelial cell cultures can be determined using assessments of cellular morphology, immunostaining of E-cadherin (an epithelial cell marker) and FSP-1 (Fibroblast Specific Protein marker). In addition, the deposition of extracellular matrix materials occurs during fibrosis development in organs such as kidneys. Based on histopathology analyses, the TDFRP compounds can decrease fibrosis in four, *in vivo* animal models of kidney injury (rat cisplatin injury model, unilateral ureteral obstruction model (UUO), nephrotoxic serum nephritis model (NTN) and streptozotocin model (STZ) of diabetic nephropathy).

Chronic kidney injury (CKI or chronic renal failure) is the gradual and irreversible loss in the ability of the kidneys to excrete wastes, concentrate urine, and conserve electrolytes, which usually progresses to end-stage renal disease (ESRD). The two most common causes of kidney injury are diabetes and hypertension. Diabetic nephropathy is a clinical syndrome characterized by albuminuria, impaired glomerular filtration rate ("GFR"), and increased risk for cardiovascular disease. In a typical experiment, TDFRP compounds can be shown to slow the progression of renal disease, repair renal fibrosis and restore renal structure using three different animal models of CKI including: Unilateral Ureteral Obstruction (UUO), a physical obstruction injury model; Nephrotoxic Serum Nephritis (NTN), an immune nephropathy injury model; and Streptozotocin-induced Diabetic Nephropathy (STZ), a metabolic model of diabetes. Each of these models induces renal injury through different mechanisms but they share downstream cellular activities that culminate in organ injury and dysfunction.

Chronic UUO is a well-accepted interstitial fibrosis model of kidney injury that is caused by the physical obstruction of the ureter. Post obstruction, there is an accumulation of immune cells and extracellular matrix materials in the kidney that lead to inflammation, interstitial fibrosis and tubular atrophy that can be alleviated by in a typical experiment using the administration of TDFRP compounds.

In a typical experiment, TDFRP compounds can limit the renal damage induced by nephrotoxic serum ("NTS") in the NTN chronic renal injury model. Most forms of glomerulonephritis are immunological in origin and the NTN model represents an antibody-induced example of glomerular disease. In a typical experiment, TDFRP compounds can diminish the amount of kidney tubular atrophy, matrix deposition/fibrosis and glomerulosclerosis in immune-mediated renal disease model.

STZ-induced Type 1 diabetes is a widely used experimental model for diabetic nephropathy. Mice given iv STZ develop a progressive loss of pancreatic beta cells culminating in diabetes and renal damage within a six month period of time. In a typical experiment, TDFRP compounds can alleviate fibrosis, reduce proteinuria and protect the kidneys from STZ damage.

Myocardial injury (MI) arises when a decrease in blood flow or obstruction in one of the major arteries feeding the heart muscle prevents oxygen and nutrients from reaching the heart muscle. The first phase of this process is an ischemic state, where decreased blood flow restricts the oxygen supply below the demand required by the heart muscle. The myocardial cells are starved for the oxygen and nutrients carried by the blood. This situation can only be sustained for very short periods of time before the cells become irreversibly injured and die. The death of these cells constitutes an infarction and the injured cells release chemicals that incite inflammatory reactions.

Myocardial ischemia (MI) leads to apoptosis, inflammation and fibrosis. TPA, administered soon after a myocardial infarction, limits damage (by reopening the blood vessel) but has no effect on the cellular processes of apoptosis, inflammation and fibrosis. Heart tissues express the same BMP-7 receptors as kidney tissues suggesting that BMP-7, with a documented role as an endogenous protective mechanism and TDFRP compounds will reduce infarction in a typical experiment involving models of myocardial ischemia.
In a typical experiment, TDFRP compounds can be used to demonstrate anti-inflammatory and anti-apoptotic activities in cultured cardiomyoctes. In a typical animal model study,

TDFRP compounds can be used to reduce the size of infarct, maintain coronary artery endothelial function and inhibit neutrophil adherence to vascular endothelium (reduced reperfusion injury) in rat models of MI. The MI rat model (called LAD occlusion model) involves the transient ligation of the left anterior descending artery to create ischemia. Upon removal of the ligature, blood flow into the heart initiates reperfusion injury, which can be monitored by perfusing the area with dye and assessing the degree of infarct. In a typical LAD experiment, TDFRP compounds can be administered before and after ischemia induced by ligation of the heart ventricle. Efficacy can be determined by morphology and by assessing CK-MB levels between infarct and non-infarct regions of the ventricle following reperfusion.

### Angiotensin-Converting Enzyme (ACE) Inhibitors

Angiotensin-Converting Enzyme (ACE) inhibitors treat hypertension by lowering arteriolar resistance and increasing venous capacity, increasing cardiac output and cardiac index, stroke work and volume, lowering renovascular resistance, and increasing excretion of sodium in the urine. According to one embodiment, ACE inhibitors include but are not limited to: sulfhydryl-containing agents such as captopril and zofenopril; dicarboxylate-containing agents such as enalapril, ramipril, quinapril, perindopril, lisinopril, and benazepril; phosphonate-containing agents such as fosinopril and ceronapril, naturally occurring ACE inhibitors such as casokinins, lactokinins; tripeptides such as Val-Pro-Pro and Ile-Pro-Pro and the nonapeptide teprotide; and other ACE inhibitors such alacepril, cilazapril, delapril imidapril moexipril, rentiapril, spirapril, temocapril, moveltipril and trandolapril.

### Neprilysin Inhibitors

Neprilysin (neutral endopeptidase, EC 3.4.24.11) (NEP), is an endothelial membrane bound Zn²⁺ metallopeptidase found in many organs and tissues, including the brain, kidneys, lungs, gastrointestinal tract, heart, and the peripheral vasculature. NEP degrades and inactivates a number of endogenous peptides, such as enkephalins, circulating bradykinin, angiotensin peptides, and natriuretic peptides, the latter of which have several effects including, for example, vasodilation and natriuresis/diuresis, as well as inhibition of cardiac hypertrophy and ventricular fibrosis. Thus, NEP plays an important role in blood pressure homeostasis and cardiovascular health.

According to some embodiments, the neprilysin inhibitor is selected from the group consisting of: thiorphan, candoxatril, and candoxatrilat.

According to other embodiments, the NEP inhibitor is a dicarboxylic acid dipeptide NEP inhibitor, described by Ksander et al. (1995) J. Med. Chem. 38:1689-1700, incorporated by reference in its entirety herein.

According to some embodiments, the additional agent is a compound that inhibits both NEP and angiotensin-I converting enzyme (ACE). Such agents include omapatrilat, gempatrilat, and sampatrilat. Referred to as vasopeptidase inhibitors, this latter class of compounds is described in Robl et al. (1999) Exp. Opin. Ther. Patents 9(12): 1665-1677, incorporated by reference in its entirety herein.

### Angiotensin Receptor-Neprilysin Inhibitors (ARNI)

Angiotensin receptor-neprilysin inhibitors (ARNIs) combine the effects of angiotensin receptor blockade with valsartan and neprilysin inhibition with sacubitril. According to one embodiment, the ARNI is sacubitril/valsartan, a combination drug that consists of the neprilysin inhibitor sacubitril and the angiotensin receptor blocker valsartan.

### METHODS

The present disclosure provides TDFRPs compounds that are functional analogs of tissue differentiation factors, *i.e.,* compounds that functionally mimic TGF-beta superfamily proteins, for example by acting as TGF-beta superfamily receptor agonists.

The disclosure provides for both prophylactic and therapeutic methods of treating a subject at risk of (or susceptible to) a disorder or having a disorder associated with aberrant TDF polypeptide or TDFRP compound target molecule expression or activity. TDF and TDFRP compound target molecules, such as TDF receptors, play a role in cell differentiation. Cell differentiation is the central characteristic of tissue morphogenesis. Tissue morphogenesis is a process involved in adult tissue repair and regeneration mechanisms. The degree of morphogenesis in adult tissue varies among different tissues and is related, among other things, to the degree of cell turnover in a given tissue.

The bone morphogenetic proteins are members of the transforming growth factor-beta superfamily. Ozkaynak et al. (EMBO J. 9: 2085-2093, 1990) purified a novel bovine osteogenic protein homolog, which they termed 'osteogenic protein-1' (OP-1; *a.k.a.,* BMP-7). The authors used peptide sequences to clone the human genomic and cDNA clones of OP-1, later named BMP-7. The BMP-7 cDNAs predicted a 431-amino acid polypeptide that includes a secretory signal sequence. The TDFRP compounds described herein are structural mimetics of the biologically active regions of bone morphogenic proteins, for example, but not limited to, BMP-7 (OP-1), and related peptides. Biologically active regions include, for example, the Finger 1 and Finger 2 regions of BMP-7. Groppe et al. (Nature 420: 636-642, 2002) reported the crystal structure of the antagonist Noggin (602991) bound to BMP-7.

TDFRP compounds are useful to treat diseases and disorders that are amenable to treatment with BMP polypeptides. According to some embodiments, the TDFRP compounds of the disclosure are useful to alter, *e.g.,* inhibit or accelerate, the ability to repair and regenerate diseased or damaged tissues and organs, as well as, to treat TDF-associated disorders. Particularly useful areas for TDFRP-based human and veterinary therapeutics include reconstructive surgery, the treatment of tissue degenerative diseases including, for example, renal disease, brain trauma, stroke, atherosclerosis, arthritis, emphysema, osteoporosis, cardiomyopathy, cirrhosis, degenerative nerve diseases, inflammatory diseases, and cancer, and in the regeneration of tissues, organs and limbs. The TDFRP compounds of the disclosure can also be used to promote or inhibit the growth and differentiation of muscle, bone, skin, epithelial, heart, nerve, endocrine, vessel, cartilage, periodontal, liver, retinal, and connective tissue, or any tissue where functional TDRFP compound target molecules are expressed. Accordingly, diseases associated with aberrant TDF polypeptide or TDFRP compound target molecule expression include viral infections, cancer, healing, neurodegenerative disorders, *e.g.,* Alzheimer's Disease, Parkinson's Disorder, immune disorders, and bone disorders. For example, TDFRP-based therapeutic compositions are useful to induce regenerative healing of bone defects such as fractures, as well as, to preserve or restoring healthy metabolic properties in diseased tissue, *e.g.,* osteopenic bone tissue.

The TDFRPs of the present disclosure are used with additional agents in various prophylactic and therapeutic methods. It is a finding of the present disclosure that by administration of a TDFRP compound with an additional agent (*e.g.* inhibitors of angiotensin converting enzyme (ACE), neprilysin inhibitors or angiotensin receptor-neprilysin inhibitors), the half-life of the TDFRP can be extended, in certain embodiments, by at least 2-10- fold. Without being bound by theory, it is thought that the combination of TDFRPs with an additional agent extends the exposure of the receptors to the TDFRPs, which allows the TDFRPs to be more effective as a therapeutic.

Accordingly, the present disclosure includes prophylactic and therapeutic methods using the TDFRPs described herein, in combination with an additional agent (e.g. inhibitors of angiotensin converting enzyme (ACE), neprilysin inhibitors or angiotensin receptor-neprilysin inhibitors).

According to some aspects, the present disclosure includes methods of modulating TDF polypeptides or TDFRP compound target molecule expression or activity in a subject for therapeutic purposes, where the TDFRP compound is administered in combination with an additional agent (e.g. inhibitors of angiotensin converting enzyme (ACE), neprilysin inhibitors or angiotensin receptor-neprilysin inhibitors). The modulatory method of the disclosure involves contacting a cell with a compound of the present disclosure, that modulates one or more of the activities of the TDF polypeptide or TDFRP compound target molecule activity associated with the cell, in combination with an additional agent (e.g. inhibitors of angiotensin converting enzyme (ACE), neprilysin inhibitors or angiotensin receptor-neprilysin inhibitors). A compound that modulates a TDF polypeptide or TDFRP compound target molecule activity is described herein, such as a nucleic acid or a polypeptide, a naturally-occurring cognate ligand of a TDFRP compound, a TDFRP compound, an anti-TDFRP compound antibody, a TDFRP compound mimetic, or a small molecule. According to one embodiment, the compound stimulates one or more TDF polypeptide or TDFRP compound target molecule activity. Examples of such stimulatory compounds include a TDFRP compound and a nucleic acid molecule encoding TDFRP compound that has been introduced into the cell. In another embodiment, the compound inhibits one or more TDF polypeptide. These modulatory methods can be performed *in vitro* (*e.g.,* by culturing the cell with the compound) or, alternatively, *in vivo* (*e.g.,* by administering the compound to a subject). As such, the disclosure provides methods of treating an individual afflicted with a TDF-associated disease or disorder characterized by aberrant expression or activity of a TDF polypeptide or TDFRP compound target molecule or nucleic acid molecules encoding them. According to one embodiment, the method involves administering a compound (*e.g.,* a compound identified by a screening assay described herein), or combination of compounds that modulates (*e.g.,* up-regulates or down-regulates) TDF polypeptide or TDFRP compound target molecule expression or activity, in combination with an additional agent (e.g. inhibitors of angiotensin converting enzyme (ACE), neprilysin inhibitors or angiotensin receptor-neprilysin inhibitors). In another embodiment, the method involves administering a TDFRP compound or nucleic acid molecule encoding TDFRP as therapy to compensate for reduced or aberrant TDF polypeptide or TDFRP compound target molecule expression or activity, in combination with an additional agent (e.g. inhibitors of angiotensin converting enzyme (ACE), neprilysin inhibitors or angiotensin receptor-neprilysin inhibitors).

Stimulation of TDF polypeptide or TDFRP compound target molecule activity is desirable *in* situations in which TDF polypeptide or TDFRP compound target molecule is abnormally downregulated and/or in which increased TDF activity is likely to have a beneficial effect. One example of such a situation is where a subject has a disorder characterized by aberrant cell proliferation and/or differentiation (*e.g.,* fibrosis).

According to one aspect, the disclosure features a method of increasing the serum half-life of a tissue differentiation factor related polypeptide (TDFRP) in a subject, the method comprising administering to the subject at least one tissue differentiation factor related polypeptide (TDFRP) in combination with an additional agent, wherein the additional agent is selected from the group consisting of an inhibitor of angiotensin converting enzyme (ACE), a neprilysin inhibitor and an angiotensin receptor-neprilysin inhibitor, wherein administration of the TDFRP and additional agent increases the serum half-life of the TDFRP compared to administration of the TDFRP alone.

According to one embodiment, the serum half-life of the TFDRP is increased when administered with the additional agent, compared to administration of the TDFRP alone. According to one embodiment, the serum half-life is increased 2-fold or more when the TDFRP is administered with the additional agent. According to one embodiment, the serum half-life is increased 5-fold or more when the TDFRP is administered with the additional agent. According to one embodiment, the serum half-life is increased 10-fold or more when the TDFRP is administered with the additional agent. According to one embodiment, the serum half-life is increased 1-fold, 2-fold, 3-fold, 4-fold, 5-fold, 6-fold, 7-fold, 8-fold, 9-fold or 10-fold or more when the TDFRP is administered with the additional agent.

According to one aspect, the disclosure features a method of preventing a tissue differentiation factor-associated disorder or disease, the method comprising administering to a subject in need of treatment at least one tissue differentiation factor related polypeptide (TDFRP) in combination with an additional agent, wherein the additional agent is selected from the group consisting of an inhibitor of angiotensin converting enzyme (ACE), a neprilysin inhibitor and an angiotensin receptor-neprilysin inhibitor, wherein the TDFRP and additional agent are administered in an amount effective to prevent the tissue differentiation factor-associated disorder or disease in the subject.

According to one aspect, the disclosure features a method of treating a tissue differentiation factor-associated disorder or disease, the method comprising administering to a subject in need of treatment at least one tissue differentiation factor related polypeptide (TDFRP) in combination with an additional agent, wherein the additional agent is selected from the group consisting of an inhibitor of angiotensin converting enzyme (ACE), a neprilysin inhibitor and an angiotensin receptor-neprilysin inhibitor, wherein the TDFRP and additional agent are administered in an amount effective to treat or prevent the tissue differentiation factor-associated disorder or disease in the subject.

According to one embodiment, the serum half-life of the TFDRP is increased when administered with the additional agent, compared to administration of the TDFRP alone. According to one embodiment, the serum half-life is increased 2-fold or more when the TDFRP is administered with the additional agent. According to one embodiment, the serum half-life is increased 5-fold or more when the TDFRP is administered with the additional agent. According to one embodiment, the serum half-life is increased 10-fold or more when the TDFRP is administered with the additional agent. According to one embodiment, the serum half-life is increased 1-fold, 2-fold, 3-fold, 4-fold, 5-fold, 6-fold, 7-fold, 8-fold, 9-fold or 10-fold or more when the TDFRP is administered with the additional agent.

According to one embodiment, the tissue differentiation factor-associated disorder is a tissue degenerative disease. According to one embodiment, the tissue differentiation factor-associated disorder is a tissue regeneration disease. According to another embodiment, the tissue regeneration disease or disorder is a disease or disorder of the kidney or renal tissue. According to one embodiment, the disease or disorder of the kidney or renal tissue is selected from the group consisting of acute kidney injury and chronic kidney disease. According to some embodiments, the tissue degenerative disease is selected from the group consisting of renal disease, macular degeneration, degenerative joint disease, traumatic brain or spinal cord injury, stroke, atherosclerosis, arthritis, emphysema, osteoporosis, cardiomyopathy, cirrhosis, degenerative nerve disease, Holt-Oram disease, eye disease, diabetic nephropathy, degenerative bone disease, liver disease, periodontal disease, diabetes, cardiovascular disease, inflammatory disease, immune disease, skeletal disease, reproductive disease, haematopoietic disease, cellular damage due to ionizing radiation, cellular damage due to hypoxia and cancer. According to other embodiments, the tissue regeneration is selected from the group consisting of muscle, dendritic tissue, nerve, kidney, brain, bone, skin, lung, muscle, ovary, testes, heart, spleen, cartilage, nerve, peridontal, dentin, liver, vascular, connective, lymphatic, haematopoietic, and renal tissue.

According to another aspect, the disclosure features a method of treating a disease or disorder associated with increased levels or biological activity of TDF polypeptides or TDFRP compound target molecules, the method comprising administering to a subject in need of treatment at least one TDFRP in combination with an additional agent, wherein the additional agent is selected from the group consisting of an inhibitor of angiotensin converting enzyme (ACE), a neprilysin inhibitor and an angiotensin receptor-neprilysin inhibitor, wherein the TDFRP and additional agent are administered in an amount effective to treat the disease or disorder associated with increased levels or biological activity of TDF polypeptides or TDFRP compound target molecules.

According to another aspect, the disclosure features a method of preventing a disease or disorder associated with increased levels or biological activity of TDF polypeptides or TDFRP compound target molecules, the method comprising administering to a subject in need of treatment at least one TDFRP in combination with an additional agent, wherein the additional agent is selected from the group consisting of an inhibitor of angiotensin converting enzyme (ACE), a neprilysin inhibitor and an angiotensin receptor-neprilysin inhibitor, wherein the TDFRP and additional agent are administered in an amount effective to prevent the disease or disorder associated with increased levels or biological activity of TDF polypeptides or TDFRP compound target molecules.

According to some embodiments, diseases and disorders that are associated with increased (relative to a subject not suffering from the disease or disorder) levels or biological activity of TDF polypeptides or TDFRP compound target molecules can be treated with TDFRP-based therapeutic compounds that antagonize (i.e., reduce or inhibit) activity, which can be administered in a therapeutic or prophylactic manner. Therapeutic compounds that can be utilized include, but are not limited to: (i) an aforementioned TDFRP compound, or analogs, derivatives, fragments or homologs thereof; (ii) anti-TDFRP compound antibodies to an aforementioned peptide; (iii) nucleic acids encoding TDFRP compound; (iv) administration of antisense nucleic acid and nucleic acids that are "dysfunctional" (i.e., due to a heterologous insertion within the coding sequences of coding sequences to a TDFRP compound) that are utilized to "knockout" endogenous function of TDFRP compound by homologous recombination (see, e.g., Capecchi, 1989. Science 244: 1288-1292); or (v) modulators (i.e., inhibitors, agonists and antagonists, including additional peptide mimetic of the disclosure or antibodies specific to a peptide of the disclosure) that alter the interaction between an aforementioned compound and its binding partner.

Diseases and disorders that are associated with decreased (relative to a subject not suffering from the disease or disorder) levels or biological activity of TDF or TDFRP compound target molecule can be treated with TDFRP-based therapeutic compounds that increase (i.e., are agonists to) TDF activity. Therapeutics that upregulate activity can be administered in a therapeutic or prophylactic manner. Therapeutics that can be utilized include, but are not limited to, TDFRP compound or analogs, derivatives, fragments or homologs thereof; or an agonist that increases bioavailability.

Increased or decreased levels can be readily detected by quantifying TDF-induced peptides and/or RNA, by obtaining a patient tissue sample (e.g., from biopsy tissue) and assaying it in vitro for RNA or peptide levels, structure and/or activity of the expressed peptides (or mRNAs of an aforementioned peptide). Methods that are well-known within the art include, but are not limited to, immunoassays (e.g., by Western blot analysis, immunoprecipitation followed by sodium dodecyl sulfate (SDS) polyacrylamide gel electrophoresis, immunocytochemistry, etc.) and/or hybridization assays to detect expression of mRNAs (e.g., Northern assays, dot blots, in situ hybridization, and the like).

According to one embodiment, the TDFRP compounds in combination with an additional agent (e.g., an inhibitor of angiotensin converting enzyme (ACE), a neprilysin inhibitor and an angiotensin receptor-neprilysin inhibitor) of the present disclosure are useful in potential prophylactic and therapeutic applications implicated in a variety of disorders in a subject including, but not limited to: those involving development, differentiation, and activation of bone cells; in diseases or pathologies of cells in blood circulation such as red blood cells and platelets; various immunological disorders and/or pathologies; autoimmune and inflammatory diseases; cardiovascular diseases; metabolic diseases; reproductive diseases, renal diseases, diabetes, brain trauma, cancer growth and metastasis; viral infections, cancer therapy, periodontal disease; tissue regeneration; acute lymphoblastic leukemia; gliomas; neurologic diseases; neurodegenerative disorders; Alzheimer's disease; Parkinson's disorder; and hematopoietic disorders

### Fibrosis

According to another aspect, the disclosure features a method of treating a disease or disorder associated with fibrosis in a subject, the method comprising administering to a subject in need of treatment at least one TDFRP in combination with an additional agent, wherein the additional agent is selected from the group consisting of an inhibitor of angiotensin converting enzyme (ACE), a neprilysin inhibitor and an angiotensin receptor-neprilysin inhibitor, wherein the TDFRP and additional agent are administered in an amount effective to treat the fibrosis in the subject.

According to another aspect, the disclosure features a method of preventing a disease or disorder associated with fibrosis in a subject, the method comprising administering to a subject in need of treatment at least one TDFRP in combination with an additional agent, wherein the additional agent is selected from the group consisting of an inhibitor of angiotensin converting enzyme (ACE), a neprilysin inhibitor and an angiotensin receptor-neprilysin inhibitor, wherein the TDFRP and additional agent are administered in an amount effective to prevent the fibrosis in the subject.

As used herein, "fibrosis" refers to the formation of excess fibrous connective tissue as a result of the excess deposition of extracellular matrix components, for example collagen. Fibrous connective tissue is characterized by having extracellular matrix (ECM) with a high collagen content. The collagen may be provided in strands or fibers, which may be arranged irregularly or aligned. The ECM of fibrous connective tissue may also include glycosaminoglycans.

According to some embodiments, treating fibrosis refers to reversing fibrosis. As used herein, "reversing fibrosis" refers to where the fibrotic material or components under treatment in a target tissue or organ is decreased or eradicated. According to certain embodiments, reversing fibrosis refers to where least about 10%, or about 25%, or about 50%, or more preferably by at least about 75%, or more preferably by about 85%, or still more preferably by about 90%, or more preferably still about by 95%, or more preferably still by 99% or more of the fibrotic components or material has been removed as compared to pretreatment. According to other embodiments, treating fibrosis refers to inhibiting fibrosis. According to certain embodiments, "inhibiting fibrosis" refers to where the net amount or level of fibrosis at a desired target fibrotic site does not increase with time. According to some embodiments, treatment of fibrosis may be effective to prevent progression of the fibrosis, *e.g.* to prevent worsening of the condition or to slow the rate of development of the fibrosis. According to some embodiments treatment or alleviation may lead to an improvement in the fibrosis, e.g. a reduction in the amount of deposited collagen fibers. Prevention of fibrosis may refer to prevention of a worsening of the condition or prevention of the development of fibrosis, e.g. preventing an early stage fibrosis developing to a later, chronic, stage.

As used herein, "excess fibrous connective tissue" refers to an amount of connective tissue at a given location (e.g. a given tissue or organ, or part of a given tissue or organ) which is greater than the amount of connective tissue present at that location in the absence of fibrosis, e.g. under normal, non-pathological conditions. As used herein, "excess deposition of extracellular matrix components" refers to a level of deposition of one or more extracellular matrix components which is greater than the level of deposition in the absence of fibrosis, e.g. under normal, non-pathological conditions.

The cellular and molecular mechanisms of fibrosis are described in Wynn, J. Pathol. (2008) 214(2): 199-210, and Wynn and Ramalingam, Nature Medicine (2012) 18:1028-1040, which are hereby incorporated by reference in their entirety.

The main cellular effectors of fibrosis are myofibroblasts, which produce a collagen-rich extracellular matrix.

In response to tissue injury, damaged cells and leukocytes produce pro-fibrotic factors such as TGFβ, IL-13 and PDGF, which activate fibroblasts to αSMA-expressing myofibroblasts, and recruit myofibroblasts to the site of injury. Myofibroblasts produce a large amount of extracellular matrix, and are important mediators in aiding contracture and closure of the wound. However, under conditions of persistent infection or during chronic inflammation there can be overactivation and recruitment of myofibroblasts, and thus overproduction of extracellular matrix components, resulting in the formation of excess fibrous connective tissue.

Diseases characterized by excessive fibrosis include but are not restricted to systemic sclerosis, scleroderma, hypertrophic cardiomyopathy, dilated cardiomyopathy (DCM), atrial fibrillation, ventricular fibrillation, myocarditis, liver cirrhosis, kidney diseases, diseases of the eye, asthma, cystic fibrosis, arthritis and idiopathic pulmonary fibrosis.

According to some embodiments, the fibrosis is selected from the group consisting of pulmonary fibrosis, renal fibrosis and hepatic fibrosis. According to one embodiment, the pulmonary fibrosis is idiopathic pulmonary fibrosis. According to another embodiment, the fibrosis is associated with a disease or condition selected from the group consisting of atherosclerosis, cardiac failure, cardiac arrhythmia, myocardial infarction, peripheral vascular disease, diabetes, chronic renal disease, pulmonary fibrosis, liver failure, and Alzheimer's disease. According to one embodiment, the disease or condition is a chronic disease or condition.

According to some embodiments fibrosis may be triggered by pathological conditions, e.g. conditions, infections or disease states that lead to production of pro-fibrotic factors such as TGFβ1. According to some embodiments, fibrosis may be caused by physical injury/stimuli, chemical injury/stimuli or environmental injury/stimuli. Physical injury/stimuli may occur during surgery, e.g. iatrogenic causes. Chemical injury/stimuli may include drug induced fibrosis, e.g. following chronic administration of drugs such as bleomycin, cyclophosphamide, amiodarone, procainamide, penicillamine, gold and nitrofurantoin (Daba et al., Saudi Med J 2004 June; 25(6): 700-6). Environmental injury/stimuli may include exposure to asbestos fibres or silica.

Fibrosis can occur in many tissues of the body. For example, fibrosis can occur in the liver (e.g. cirrhosis), lungs, kidney, heart, blood vessels, eye, skin, pancreas, intestine, brain, and bone marrow. Fibrosis may also occur in multiple organs at once.

According to some embodiments, fibrosis may involve an organ of the gastrointestinal system, e.g. of the liver, small intestine, large intestine, or pancreas. According to some embodiments, fibrosis may involve an organ of the respiratory system, e.g. the lungs. According to embodiments, fibrosis may involve an organ of the cardiovascular system, e.g. of the heart or blood vessels. According to some embodiments, fibrosis may involve the skin. According to some embodiments, fibrosis may involve an organ of the nervous system, e.g. the brain. According to some embodiments, fibrosis may involve an organ of the urinary system, e.g. the kidneys. According to some embodiments, fibrosis may involve an organ of the musculoskeletal system, e.g. muscle tissue.

According to one embodiment, treating fibrosis comprises restoring function to the tissue that is affected. For example, according to one embodiment, treating the pulmonary fibrosis comprises restoring the function of the pulmonary tissue. According to one embodiment, treating the renal fibrosis comprises restoring the function of the renal tissue. According to one embodiment, treating the hepatic fibrosis comprises restoring the function of the hepatic tissue. Any diagnostic test known in the art can be used to determine normal function of the tissue. For example, a liver (hepatic) function panel can be used to check how well the liver is working. This test measures the blood levels of total protein, albumin, bilirubin, and liver enzymes. Tests to measure lung volume, capacity, rates of flow, and gas exchange can be used to determine pulmonary function. Glomerular Filtration Rate(GFR) can be used to measure of how well the kidneys are removing wastes and excess fluid from the blood.

According to some embodiments, the fibrosis is cardiac or myocardial fibrosis, hepatic fibrosis, or renal fibrosis. According to some embodiments cardiac or myocardial fibrosis is associated with dysfunction of the musculature or electrical properties of the heart, or thickening of the walls of valves of the heart. According to some embodiments fibrosis is of the atrium and/or ventricles of the heart. Treatment or prevention of atrial or ventricular fibrosis may help reduce risk or onset of atrial fibrillation, ventricular fibrillation, or myocardial infarction.

According to some embodiments, hepatic fibrosis is associated with chronic liver disease or liver cirrhosis. According to some preferred embodiments renal fibrosis is associated with chronic kidney disease.

According to other embodiments, diseases/conditions associated with fibrosis include, but are not limited to respiratory conditions such as pulmonary fibrosis, cystic fibrosis, idiopathic pulmonary fibrosis, progressive massive fibrosis, scleroderma, obliterative bronchiolitis, Hermansky-Pudlak syndrome, asbestosis, silicosis, chronic pulmonary hypertension, AIDS associated pulmonary hypertension, sarcoidosis, tumor stroma in lung disease, and asthma; chronic liver disease, primary biliary cirrhosis (PBC), schistosomal liver disease, liver cirrhosis; cardiovascular conditions such as hypertrophic cardiomyopathy, dilated cardiomyopathy (DCM), fibrosis of the atrium, atrial fibrillation, fibrosis of the ventricle, ventricular fibrillation, myocardial fibrosis, Brugada syndrome, myocarditis, endomyocardial fibrosis, myocardial infarction, fibrotic vascular disease, hypertensive heart disease, arrhythmogenic right ventricular cardiomyopathy (ARVC), tubulointerstitial and glomerular fibrosis, atherosclerosis, varicose veins, cerebral infarcts; neurological conditions such as gliosis and Alzheimer's disease; muscular dystrophy such as Duchenne muscular dystrophy (DMD) or Becker's muscular dystrophy (BMD); gastrointestinal conditions such as Crohn's disease, microscopic colitis and primary sclerosing cholangitis (PSC); skin conditions such as scleroderma, nephrogenic systemic fibrosis and cutis keloid; arthrofibrosis; Dupuytren's contracture; mediastinal fibrosis; retroperitoneal fibrosis; myelofibrosis; Peyronie's disease; adhesive capsulitis; kidney disease (e.g., renal fibrosis, nephritic syndrome, Alport's syndrome, HIV associated nephropathy, polycystic kidney disease, Fabry's disease, diabetic nephropathy, chronic glomerulonephritis, nephritis associated with systemic lupus); progressive systemic sclerosis (PSS); chronic graft versus host disease; diseases of the eye such as Grave's ophthalmopathy, epiretinal fibrosis, retinal fibrosis, subretinal fibrosis (e.g. associated with macular degeneration (e.g. wet age-related macular degeneration (AMD)), diabetic retinopathy, glaucoma, corneal fibrosis, post-surgical fibrosis (e.g. of the posterior capsule following cataract surgery, or of the bleb following trabeculectomy for glaucoma), conjunctival fibrosis, subconjunctival fibrosis; arthritis; fibrotic pre-neoplastic and fibrotic neoplastic disease; and fibrosis induced by chemical or environmental insult (e.g., cancer chemotherapy, pesticides, radiation/cancer radiotherapy).

It will be appreciated that the many of the diseases/conditions listed above are interrelated. For example, fibrosis of the ventricle may occur post myocardial infarction, and is associated with DCM, HCM and myocarditis.

According to some embodiments, the disease/condition associated with fibrosis is idiopathic pulmonary fibrosis (IPF). IPF is a debilitating and life-threatening lung disease characterized by a progressive scarring of the lungs that hinders oxygen uptake. The cause of IPF is not known. As scarring progresses, patients with IPF experience shortness of breath (dyspnea) and difficulty with performing routine functions, such as activities of daily living. According to the National Institutes of Health, about 100,000 people in the United States have IPF, and approximately 30,000 to 40,000 new cases are found each year. Worldwide, IPF affects 13 to 20 out of every 100,000 people. A similar prevalence exists for six other interstitial lung diseases and systemic sclerosis that may benefit from antifibrotic therapy. There are no FDA-approved treatments for IPF, and approximately two-thirds of patients die within five years after diagnosis. Patients are often treated with corticosteroids and immunosuppressive agents; however, none have been clinically proven to improve survival or quality of life. It is thought that stabilization or reversal of lung fibrosis could stabilize lung function and diminish the impact of this devastating disease. Thus, the TDFRP compounds in combination with an additional agent (e.g., an inhibitor of angiotensin converting enzyme (ACE), a neprilysin inhibitor and an angiotensin receptor-neprilysin inhibitor) of the present disclosure may be used to inhibit or reverse lung fibrosis associated with IPF.

According to some embodiments, the disease/disorder may be one of pulmonary fibrosis, atrial fibrillation, ventricular fibrillation, hypertrophic cardiomyopathy (HCM), dilated cardiomyopathy (DCM), non-alcoholic steatohepatitis (NASH), cirrhosis, chronic kidney disease, scleroderma, systemic sclerosis, keloid, cystic fibrosis, Chron's disease, post-surgical fibrosis or retinal fibrosis.

While remarkable progress has been made in the ability to transplant various organs, long term preservation (greater than one year) of organ function and patient survival suffers in a large part because of chronic rejection. The precise manifestations of chronic rejection vary according to the transplanted organ, but all exhibit proliferation of myofibroblasts, or related cells, ultimately resulting in fibrosis that leads to loss of function. At this time, no drugs are available for treatment of the fibroproliferative lesions of progressive chronic allograft rejection. Thus, according to other aspects, the disclosure features a method of treating fibrosis associated with organ transplants, the method comprising administering to a subject in need of treatment at least one TDFRP in combination with an additional agent, wherein the additional agent is selected from the group consisting of an inhibitor of angiotensin converting enzyme (ACE), a neprilysin inhibitor and an angiotensin receptor-neprilysin inhibitor. According to one embodiment, the transplanted organ is the heart. According to one embodiment, the transplanted organ is the kidney. According to one embodiment, the transplanted organ is the liver. According to one embodiment, the transplanted organ is the lung.

### Cancer

According to certain embodiments, TDFRPs and additional agents (e.g., an inhibitor of angiotensin converting enzyme (ACE), a neprilysin inhibitor and an angiotensin receptor-neprilysin inhibitor) can be used to treat cancer. The present disclosure is not limited to a certain type of cancer, but rather any cancer is contemplated as being treated by the TDFRPs and additional agents (e.g., an inhibitor of angiotensin converting enzyme (ACE), a neprilysin inhibitor and an angiotensin receptor-neprilysin inhibitor) described herein.

According to certain embodiments, the cancer includes, but is not limited to, a cancer selected from acute lymphoblastic leukemia (ALL), ACUTE myeloid leukemia (AML), anal cancer, bile duct cancer, bladder cancer, bone cancer, bowel cancer, brain tumors, breast cancer, cancer of unknown primary, cancer spread to bone, cancer spread to brain, cancer spread to liver, cancer spread to lung, carcinoid, cervical cancer, choriocarcinoma, chronic lymphocytic leukemia (CLL), chronic myeloid leukemia (CML), colon cancer, colorectal cancer, endometrial cancer, eye cancer, gallbladder cancer, gastric cancer, gestational trophoblastic tumors (GTT), hairy cell leukemia, head and neck cancer, Hodgkin lymphoma, kidney cancer, laryngeal cancer, leukemia, liver cancer, lung cancer, lymphoma, melanoma skin cancer, mesothelioma, men's cancer, molar pregnancy, mouth and oropharyngeal cancer, myeloma, nasal and sinus cancers, nasopharyngeal cancer, non Hodgkin lymphoma (NHL), oesophageal cancer, ovarian cancer, pancreatic cancer, penile cancer, prostate cancer, rare cancers, rectal cancer, salivary gland cancer, secondary cancers, skin cancer (non melanoma), soft tissue sarcoma, stomach cancer, testicular cancer, thyroid cancer, unknown primary cancer, uterine cancer, vaginal cancer, and vulval cancer.

According to one embodiment, the cancer is prostate cancer. According to one embodiment, the cancer is breast cancer. According to one embodiment, the cancer is glioblastoma.

### Central Nervous System Injury

According to some embodiments, TDFRPs in combination with an additional agent (e.g., an inhibitor of angiotensin converting enzyme (ACE), a neprilysin inhibitor and an angiotensin receptor-neprilysin inhibitor) can be used to treat or prevent one or more adverse consequences of central nervous system injury that arise from a variety of conditions. The term "stroke" connotes central nervous system injury resulting from sudden and dramatic neurologic deficits associated with, *e.g.,* but not limited to, thrombus, embolus, and systemic hypotension. Other injuries may be caused by hypertension, hypertensive cerebral vascular disease, rupture of an aneurysm, an angioma, blood dyscrasia, cardiac failure, cardiac arrest, cardiogenic shock, kidney failure, septic shock, head trauma, spinal cord trauma, seizure, bleeding from a tumor, or other loss of blood volume or pressure. These injuries lead to disruption of physiologic function, subsequent death of neurons, and necrosis (infarction) of the affected areas.

The TDFRPs in combination with an additional agent (e.g., an inhibitor of angiotensin converting enzyme (ACE), a neprilysin inhibitor and an angiotensin receptor-neprilysin inhibitor) can be used to of the present disclosure can be useful for treating traumatic injuries to the central nervous system that are caused by mechanical forces, such as a blow to the head. Such traumatic tissue insults may involve, *e.g.,* but not limited to, an abrasion, incision, contusion, puncture, compression. Traumatic injuries to the central nervous system can arise from traumatic contact of a foreign object with any locus of or appurtenant to the mammalian head, neck or vertebral column. Other forms of traumatic injury can arise from constriction or compression of mammalian CNS tissue by an inappropriate accumulation of fluid *(e.g.,* a blockade or dysfunction of normal cerebrospinal fluid or vitreous humor fluid production, turnover or volume regulation, or a subdural or intracranial hematoma or edema). Similarly, traumatic constriction or compression can arise from the presence of a mass of abnormal tissue, such as a metastatic or primary tumor.

### Cardiovascular Diseases or Disorders

According to some embodiments, TDFRPs in combination with an additional agent (e.g., an inhibitor of angiotensin converting enzyme (ACE), a neprilysin inhibitor and an angiotensin receptor-neprilysin inhibitor) can be used treat or prevent a disease or disorder of the heart or cardiovascular system. According to one embodiment, treating the disease or disorder of the heart or cardiovascular system comprises restoring the function of the heart or cardiovascular system. Heart function can be determined using diagnostic methods known in the art, including, but not limited to, electrocardiogram (ECG), echocardiogram or a stress test. According to one embodiment, the disease or disorder of the heart of cardiovascular system is selected from the group consisting of pulmonary artery hypertension, acute myocardial infarction, chronic congestive heart failure, cardiomyopathy and coronary vasculopathy. According to some embodiments, TDFRPs in combination with an additional agent (e.g., an inhibitor of angiotensin converting enzyme (ACE), a neprilysin inhibitor and an angiotensin receptor-neprilysin inhibitor) can be used treat or prevent coronary atherosclerosis.

### Hepatic Diseases or Disorders

According to some embodiments, TDFRPs in combination with an additional agent (e.g., an inhibitor of angiotensin converting enzyme (ACE), a neprilysin inhibitor and an angiotensin receptor-neprilysin inhibitor) can be used to treat or prevent a disease or disorder of the liver or hepatic system. According to one embodiment, the disease or disorder of the liver or hepatic system is liver failure. According to one embodiment, treating the disease or disorder of the liver or hepatic system comprises restoring the function of the liver or hepatic system.

### Renal Diseases or Disorders

According to some embodiments, TDFRPs in combination with an additional agent (e.g., an inhibitor of angiotensin converting enzyme (ACE), a neprilysin inhibitor and an angiotensin receptor-neprilysin inhibitor) can be used to treat or prevent renal dysfunction, disease and injury, e.g. ureteral obstruction, acute and chronic renal failure, renal fibrosis, and diabetic nephropathy. According to one embodiment, treating the renal dysfunction, disease and injury comprises restoring the function of the renal tissue.

Alport syndrome is a genetic disorder resulting from mutations in type IV collagen genes. The defect results in pathological changes in kidney glomerular and inner-ear basement membranes. In the kidney, progressive glomerulonephritis culminates in tubulointerstitial fibrosis and death. Using gene knockout-mouse models, it has been shown that two different pathways, one mediated by transforming growth factor (TGF)-β 1 and the other by integrin alpha1beta1, affect Alport glomerular pathogenesis in distinct ways. The mRNAs encoding TGF- β1 (in both mouse and human), entactin, fibronectin, and the collagen alpha1(IV) and alpha2(IV) chains are significantly induced in total kidney as a function of Alport renal disease progression. The induction of these specific mRNAs is observed in the glomerular podocytes of animals with advanced disease. Type IV collagen, laminin-1, and fibronectin are shown to be elevated in the tubulointerstitium at 10 weeks, but not at 6 weeks, suggesting that elevated expression of specific mRNAs on Northern blots reflects events associated with tubulointerstitial fibrosis. Thus, concomitant accumulation of mRNAs encoding TGF- β1 and extracellular matrix components in the podocytes of diseased kidneys may reflect key events in Alport renal disease progression. Importantly, TGF- β1 appears to have a critical role in both glomerular and tubulointerstitial damage associated with Alport syndrome. Recently, BMP-7 has been implicated to reverse the renal pathology caused by TGF-β in Alport renal disease progress. BMP-7 inhibits tubular epithelial cell de-differentiation, mesenchymal transformation and apoptosis stimulated by various renal injuries. Also it preserves glomerular integrity and inhibits injury-mediated mesangial matrix accumulation. BMP-7 may be a powerful new therapeutic agent for chronic kidney disease, with the novel attribute of not only treating the kidney disease itself, but also directly inhibiting some of the most important complications of the Alport syndrome.

### Tissue Repair

According to some embodiments, TDFRPs in combination with an additional agent (e.g., an inhibitor of angiotensin converting enzyme (ACE), a neprilysin inhibitor and an angiotensin receptor-neprilysin inhibitor) can be used to facilitate tissue repair.

### Other Diseases or Disorders

TDFRP compounds can be used to in the prophylaxis or treatment of diseases of the oral cavity, *e.g.,* by affecting direct capping of bioactive molecules, or inducing the formation of reparative dentin and coronal or radicular pulp mineralization (Goldberg et al., Am J Dent. 2003 Feb; 16(1):66-76). Further, TDFRP can be used in the prophylaxis or treatment of periodontal disease. TDFRP compounds can be used in bone tissue engineering. Lu et al., (Biochem Biophys Res Commun. 2003 Jun 13; 305(4):882-90 have shown the efficacy of a BMP-polymer matrix in inducing the expression of the osteoblastic phenotype by muscle-derived cells and present a new paradigm for bone tissue engineering. TDFRP compounds may be used in bone transplantation (Rees and Haddad, Hosp Med. 2003 Apr; 64(4):205-9). TDFRP can also be used to promote bone healing. Maniscalco et al., (Acta Biomed Ateneo Parmense. 2002; 73(1-2):27-33) verify the therapeutic potential of this BMP-7 protein in fresh tibial closed fractures, using BMP-7 associated with osteosynthesis by means of a monolateral external fixator. Moreover, TDFRP compounds can be used in the regeneration of bone tissue, *e.g.,* reconstructive surgery of the hip. Cook et al., (J Arthroplasty. 2001 Dec; 16(8 Suppl 1):88-94) demonstrated that the use of BMP-7 in conjunction with morcellized cancellous bone and cortical strut allograft in preclinical models dramatically improved the biologic activity of the graft, resulting in greater and earlier new bone formation and graft incorporation. The clinical use of BMP-7 in hip reconstructive procedures also resulted in greater and earlier new bone formation in the more challenging biologic environment compared with allograft bone alone.

TDFRP compounds can be used to treat skeletal defects e.g., acquired and congenital skeletal defects arise from trauma and developmental abnormalities as well as ablative cancer surgery. Rutherford et al., (Drug News Perspect. 2003 Jan-Feb; 16(1):5-10) discusses recent advances in bone morphogenetic protein 7 *ex vivo* gene therapy for localized skeletal regeneration address these limitations.

TDFRP compounds can be used in the prophylaxis or treatment of disorders of haematopoiesis. Studies by Detmer and Walker (Cytokine. 2002 Jan 7; 17(1):36-42) indicate that individual BMPs form part of the complement of cytokines regulating the development of haematopoietic progenitors, and in particular, point to a role for BMP-4 in the control of definitive, as well as embryonic erythropoiesis. TDFRP compounds have been demonstrated to modulate cytokine production in various cell populations (*e.g.* HK-2 cell lines, cardiomyocytes, kidney tissues), repair kidney damage and/or protect kidneys from injury, which may affect the regulation and production of hematopoietic progenitor cells and growth factors (FIG. 14).
TDFRP compounds can be used in the treatment of reproductive disorders, *e.g.,* sterility. Zhao et al., (Dev Biol. 2001 Dec 1; 240(1):212-22) demonstrated that mutation in BMP-7 exacerbates the phenotype of BMP-8a mutants in spermatogenesis and epididymis. These indicate that, similar to BMP-8a, BMP-7 plays a role in both the maintenance of spermatogenesis and epididymal function and it further suggests that BMP-8 and BMP-7 signal through the same or similar receptors in these two systems.

### Biological Effect

In various embodiments of the disclosure, suitable *in vitro* or *in vivo* assays are performed to determine the effect of a specific TDFRP-based therapeutic in combination with an additional agent (e.g., an inhibitor of angiotensin converting enzyme (ACE), a neprilysin inhibitor and an angiotensin receptor-neprilysin inhibitor), and whether its administration is indicated for treatment of the affected tissue in a subject.

In various specific embodiments, *in vitro* assays can be performed with representative cells of the type(s) involved in the patient's disorder, to determine if a given TDFRP-based therapeutic in combination with an additional agent (e.g., an inhibitor of angiotensin converting enzyme (ACE), a neprilysin inhibitor and an angiotensin receptor-neprilysin inhibitor) exerts the desired effect upon the cell type(s). Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art can be used prior to administration to human subjects.

Specific *in vivo* assays for testing the efficacy of a compound or analog, *e.g.,* TDFRP compound, in combination with an additional agent (e.g., an inhibitor of angiotensin converting enzyme (ACE), a neprilysin inhibitor and an angiotensin receptor-neprilysin inhibitor), in an application to repair or regenerate damaged bone, liver, kidney, or nerve tissue, periodontal tissue, including cementum and/or periodontal ligament, gastrointestinal and renal tissues, and immune-cell mediated damages tissues are disclosed in publicly available documents, which include, for example, EP 0575,555; WO93/04692; WO93/05751; WO/06399; WO94/03200; WO94/06449; and WO94/06420, each incorporated herein by reference in their entireties.

### In Vivo Functional Assays

### Rat Cisplatin Model of Acute Renal Injury:

The rat cisplatin model has been studied extensively and is considered a relevant model for human acute kidney injury. Kidneys from cisplatin-treated animals show little evidence of cell proliferation. In contrast, animals that are protected or are able to recover from the nephrotoxic effects of cisplatin treatment show proliferation as detected by anti-proliferating cell nuclear antigen or PCNA antibodies. This model provides a mechanism for identifying agents that can protect the kidney from inflammatory cytokine-mediated injury, as well as, in repairing organ damage. Macrophages infiltrate the kidneys of animals treated with cisplatin and this early marker of nephropathy can be detected using anti CD-68 antibodies. Agents that decrease the number of macrophages in kidneys and prevent inflammatory cell-mediated damage associated with cisplatin treatment can be detected using immuno-histological analysis. Kidney sections treated with agents that show increased expression of α-actin in smooth muscle cells (SMC) are indicative of protecting the integrity of SMC and alleviating injurious damage to the kidney.

Male Sprague-Dawley rats (~250 gm) are injected *ip* with 5 mg/kg cisplatin (1 mg/ml in water) at time zero to induce acute renal failure. In this model, clinical signs (rise in serum creatinine) peaks at 4 to 5 days. The test compound and control substances were administered iv via the tail vein as 0.5 ml slow bolus injections. The negative control substance was the vehicle (PBS pH 7.2 + 5% Mannitol), while the positive control therapy was recombinant human mature BMP-7 (BMP-7 or TDF-1). The end points are serum creatinine and BUN levels and histology.

### Unilateral Ureteral Obstruction Model of Chronic Renal Injury:

Unilateral ureteral obstruction (UUO) is a model resembling human obstructive nephropathy, which represents an inducible chronic model of interstitial fibrosis. UUO is induced by ligation of one ureter, leaving the contra-lateral kidney to serve as a control. As a result of the procedure, mice develop renal tubulointerstitial inflammation, tubulointerstitial fibrosis and tubular atrophy, without hypertension, proteinuria, lipid dysregulation and little glomerular damage contributing to the disease state (Ito et al (2004).J Urol. Feb; 171(2 Pt 1): 926-30.; Bhangdia et al. (2003) J Urol. Nov; 170(5): 2057-62.; Rawashdeh et al. (2003) Invest Radiol. 2003 Mar; 38(3): 153-8.; Hruska et al (2000) Am J Physiol Renal Physiol. 2000 Jul; 79(1): F130-43.). Furthermore, renal fibrosis is associated with alteration of epithelial and fibroblastic cells to myofibroerblasts, along with increased expression of TGF-beta and progressive loss of renal function.

### Nephrotoxic Serm Nephritis (NTN) Model of Chronic Kidney Injury:

Renal fibrosis involves interestitial fibrosis, tubular atrophy, and glomerulosclerosis. The mouse nephrotoxic serum nephritis (NTN) renal fibrosis model mimics human chronic renal injury. NTN mice develop glomerulonephritis following injection with nephrotoxic serum (NTS), which progresses to tubulointerstitial disease and eventually renal fibrosis after 6 weeks (see Lloyd, et al. (1997) J. Exp. Med. 185, 1371-1380.; Zeisberg et al. (2003) Nat. Med. 9, 964-968.)

### PHARMACEUTICAL COMPOSITIONS

The pharmaceutical compositions of the disclosure typically contain a therapeutically effective amount of a compound described herein. Those skilled in the art will recognize, however, that a pharmaceutical composition may contain more than a therapeutically effective amount, such as in bulk compositions, or less than a therapeutically effective amount, that is, individual unit doses designed for multiple administration to achieve a therapeutically effective amount. Typically, the composition will contain from about 0.01-95 wt % of active agent, including, from about 0.01-30 wt %, such as from about 0.01-10 wt %, with the actual amount depending upon the formulation itself, the route of administration, the frequency of dosing, and so forth. According to one embodiment, a composition suitable for an oral dosage form, for example, may contain about 5-70 wt %, or from about 10-60 wt % of active agent.

According to some aspects, the TDFRP compounds of the disclosure, and derivatives, fragments, analogs and homologs thereof, and additional agent are be incorporated into pharmaceutical compositions suitable for administration.

TDFPR compounds of the disclosure may be physically mixed with the additional agent to form a composition containing both agents; or the TDFRP and additional agent each may be present in separate and distinct compositions which are administered to the patient simultaneously or at separate times. For example, a TDFRP compound of the disclosure can be combined with an additional agent using conventional procedures and equipment to form a combination of active agents comprising a TDFRP compound of the disclosure and an additional agent. Additionally, the additional agents may be combined with a pharmaceutically acceptable carrier to form a pharmaceutical composition comprising a compound of the disclosure, a second active agent and a pharmaceutically acceptable carrier. In this embodiment, the components of the composition are typically mixed or blended to create a physical mixture. The physical mixture is then administered in a therapeutically effective amount using any of the routes described herein.

Alternatively, the TDFRP compound and additional agent may remain separate and distinct before administration to the patient. In this embodiment, the TDFRP compound and additional agent are not physically mixed together before administration but are administered simultaneously or at separate times as separate compositions. Such compositions can be packaged separately or may be packaged together in a kit. When administered at separate times, the additional agent will typically be administered less than 24 hours after administration of the compound of the disclosure, ranging anywhere from concurrent with administration of the compound of the disclosure to about 24 hours post-dose. This is also referred to as sequential administration. Thus, a compound of the disclosure can be orally administered simultaneously or sequentially with the additional agent using two tablets, with one tablet for the TDFRP compound and one tablet for the additional agent, where sequential may mean being administered immediately after administration of the compound of the disclosure or at some predetermined time later (for example, one hour later or three hours later). It is also contemplated that the additional agent may be administered more than 24 hours after administration of the compound of the disclosure. Alternatively, the combination may be administered by different routes of administration, that is, one orally and the other by inhalation.

According to one embodiment, the TDFRP is provided in the same pharmaceutical composition as the additional agent. According to another embodiment, the TDFRP is provided in a separate composition as the additional agent.

As used herein, "pharmaceutically acceptable carrier" is intended to include any and all solvents, dispersion media, coatings, antibacterial and antifungal compounds, isotonic and absorption delaying compounds, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, Ringer's solutions, dextrose solution, and 5% human serum albumin. Liposomes and nonaqueous vehicles such as fixed oils may also be used. The use of such media and compounds for pharmaceutically active substances is well known in the art. Except insofar as any conventional media or compound is incompatible with the active compound, use thereof in the compositions is contemplated. Supplementary active compounds can also be incorporated into the compositions.

A pharmaceutical composition of the disclosure is formulated to be compatible with its intended route of administration. Examples of routes of administration include parenteral, *e.g.,* intravenous, intradermal, subcutaneous, oral (*e.g.,* inhalation), transdermal (*i.e.,* topical), transmucosal, and rectal administration. According to some embodiments, a pharmaceutical composition is formulated to be compatible with intravenous, intraperitoneal, intramuscular, subcutaneous, inhalation, transmucosal, and oral routes of administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial compounds such as benzyl alcohol or methyl parabens; antioxidants such as ascorbic acid or sodium bisulfite; chelating compounds such as ethylenediaminetetraacetic acid (EDTA); buffers such as acetates, citrates or phosphates, and compounds for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal compounds, for example, parabens, chlorobutanol, phenol, ascorbic acid, thimerosal, and the like. In many cases, it will be preferable to include isotonic compounds, for example, sugars, polyalcohols such as manitol, sorbitol, sodium chloride in the composition. Prolonged absorption of the injectable compositions can be brought about by including in the composition a compound, which delays absorption, for example, aluminum monostearate and gelatin.

Sterile injectable solutions can be prepared by incorporating the active compound (*e.g.,* a TDFRP compound and/or additional agent) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding compounds, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating compound such as alginic acid, Primogel, or corn starch; a lubricant such as magnesium stearate or Sterotes; a glidant such as colloidal silicon dioxide; a sweetening compound such as sucrose or saccharin; or a flavoring compound such as peppermint, methyl salicylate, or orange flavoring.

Release agents, wetting agents, coating agents, sweetening, flavoring and perfuming agents, preservatives and antioxidants may also be present in the pharmaceutical compositions. Exemplary coating agents for tablets, capsules, pills and like, include those used for enteric coatings, such as cellulose acetate phthalate, polyvinyl acetate phthalate, hydroxypropyl methylcellulose phthalate, methacrylic acid-methacrylic acid ester copolymers, cellulose acetate trimellitate, carboxymethyl ethyl cellulose, hydroxypropyl methyl cellulose acetate succinate, and the like. Examples of pharmaceutically acceptable antioxidants include: water-soluble antioxidants, such as ascorbic acid, cysteine hydrochloride, sodium bisulfate, sodium metabisulfate sodium sulfite and the like; oil-soluble antioxidants, such as ascorbyl palmitate, butylated hydroxyanisole, butylated hydroxytoluene, lecithin, propyl gallate, alpha-tocopherol, and the like; and metal-chelating agents, such as citric acid, ethylenediamine tetraacetic acid, sorbitol, tartaric acid, phosphoric acid, and the like.

For administration by inhalation, the compounds are delivered in the form of an aerosol spray from pressured container or dispenser, which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared as pharmaceutical compositions in the form of suppositories (*e.g*., with conventional suppository bases such as cocoa butter and other glycerides) or retention enemas for rectal delivery. The compounds can be prepared for use in conditioning or treatment of *ex vivo* explants or implants.

Compositions may also be formulated to provide slow or controlled release of the active agent using, by way of example, hydroxypropyl methyl cellulose in varying proportions or other polymer matrices, liposomes and/or microspheres. In addition, the pharmaceutical compositions of the disclosure may contain opacifying agents and may be formulated so that they release the active agent only, or preferentially, in a certain portion of the gastrointestinal tract, optionally, in a delayed manner. Examples of embedding compositions which can be used include polymeric substances and waxes. The active agent can also be in micro-encapsulated form, optionally with one or more of the above-described excipients. According to one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

According to some embodiments, it is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved, and the limitations inherent in the art of compounding such an active compound for the treatment of individuals.

The nucleic acid molecules of the disclosure can be inserted into vectors and used as gene therapy vectors. Gene therapy vectors can be delivered to a subject by, for example, intravenous injection, local administration (*see, e.g.,* U.S. Pat. No. 5,328,470) or by stereotactic injection (*see, e.g.,* Chen, et al., 1994. Proc. Natl. Acad. Sci. USA 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g*., retroviral vectors, the pharmaceutical preparation can include one or more cells that produce the gene delivery system. The pharmaceutical compositions can be included in a container, pack, or dispenser together with instructions for administration.

All publications and patent applications cited in this specification are herein incorporated by reference in their entirety for all purposes as if each individual publication or patent application were specifically and individually indicated to be incorporated by reference for all purposes. The publications discussed herein are provided solely for their disclosure prior to the filing date of the present application. Nothing herein is to be construed as an admission that the inventors described herein are not entitled to antedate such disclosure by virtue of prior disclosure or for any other reason.

### EXAMPLES

The following examples are intended to be non-limiting illustrations of certain embodiments of the present disclosure. All references cited are hereby incorporated herein by reference in their entireties.

### Example 1. Combination of TDFRPs and ACE Inhibitor increases TDFRP Serum half-life

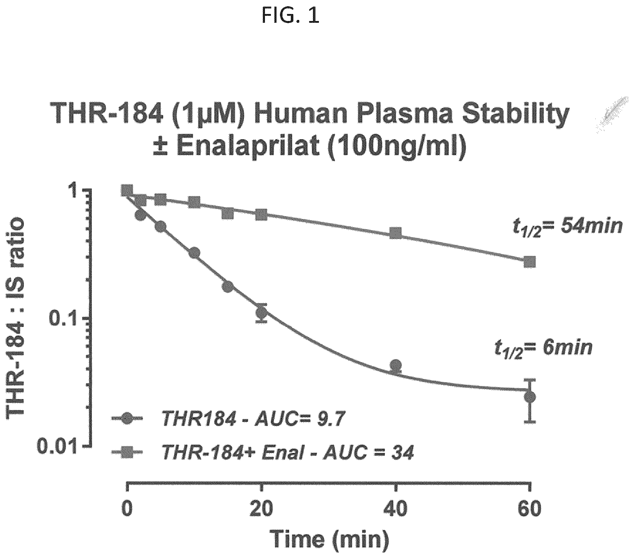

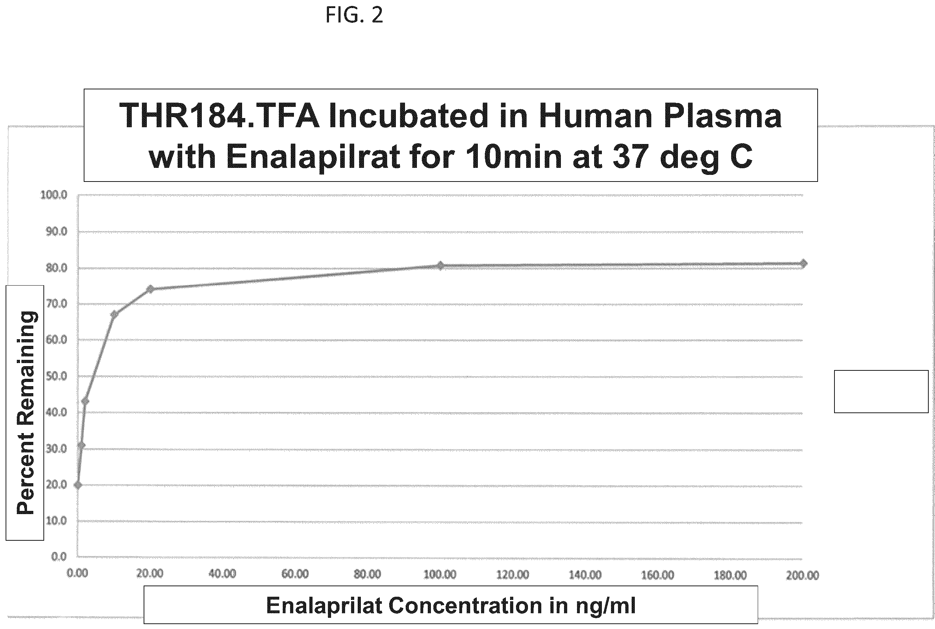

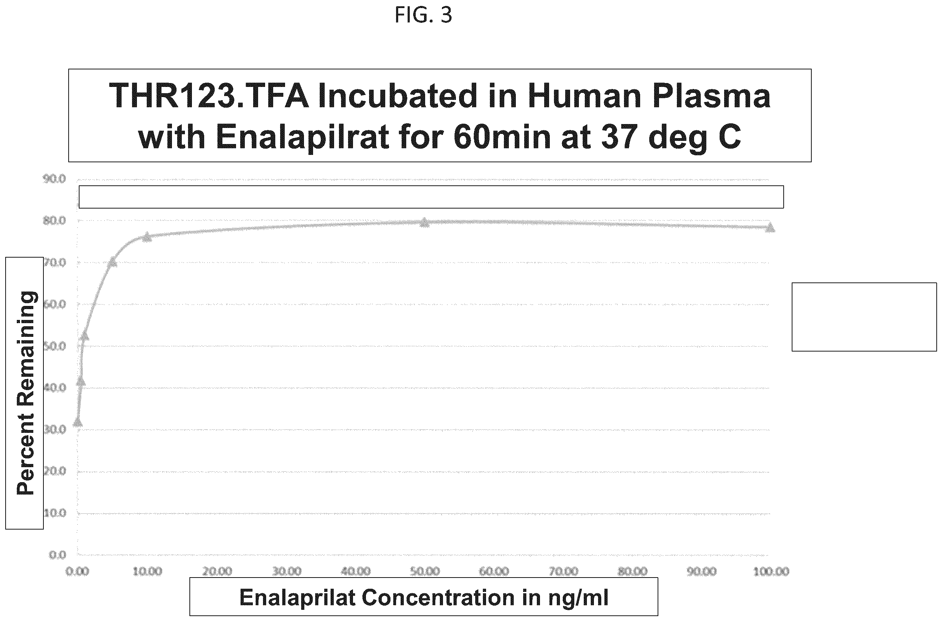

TDFRPs have been found to have a short serum half-life with large variability in AUC and peak serum concentrations in humans. In a set of experiments, various TDFRPs were incubated with ACE inhibitors and the percent TDFRP remaining in human plasma was determined. FIG. 1 is a graph that shows the human plasma stability over time of tissue differentiation factor related polypeptide (TDFRP) THR-184 alone or in combination with Enalaprilat. FIG. 2 is a graph that shows the percent of TDFRP polypeptide THR-184 (1000 ng/ml) remaining in human plasma after incubation with Lisinopril or Enalaprilat for 10 minutes at 37°C. Concentration of Enalaprilat is shown on the x-axis (mg/ml). FIG. 3 is a graph that shows the percent of TDFRP polypeptides THR-184.TFA (trifluoroacetic acid) and THR-204.AC (acetic acid) (1000 ng/ml) remaining in human plasma after incubation with Enalaprilat for 60 minutes at 37°C. Concentration of Enalaprilat is shown on the x-axis (mg/ml). By adding an ACE inhibitor, the serum half-life of the TDFRPs tested was extended by ten fold from 5 min to 50 min. These experiments show that the concomitant administration of the TDFRPs with an ACE inhibitor extended the exposure of the receptors to the compound, and thus the TDFRPs will be more effective as a therapeutic.
The present disclosure will now be further described, by way of example only, with reference to the following clauses:
Clause 1. A method of treating or preventing a tissue differentiation factor-associated disorder or disease, the method comprising: administering to a subject in need of treatment at least one tissue differentiation factor related polypeptide (TDFRP) in combination with an additional agent, wherein the additional agent is selected from the group consisting of: an inhibitor of angiotensin converting enzyme (ACE), a neprilysin inhibitor and an angiotensin receptor-neprilysin inhibitor, wherein the TDFRP and additional agent are administered in an amount effective to treat or prevent the tissue differentiation factor-associated disorder or disease in the subject.
Clause 2. The method of clause 1, wherein the serum half-life of the TFDRP is increased when administered with the additional agent, compared to administration of the TDFRP alone.
Clause 3. The method of clause 2, wherein serum half-life is increased between about 5-fold to about 10-fold or more when the TDFRP is administered with the additional agent.
Clause 4. The method of clause 1, wherein the tissue differentiation factor-associated disorder is selected from a tissue degenerative disease or a tissue regeneration disease.
Clause 5. The method of clause 4, wherein the tissue regeneration disease or disorder is a disease or disorder of the kidney or renal tissue.
Clause 6. The method of clause 5, wherein the disease or disorder of the kidney or renal tissue is selected from the group consisting of: acute kidney injury and chronic kidney disease.
Clause 7. The method of clause 5, wherein treating the disease or disorder of the kidney or renal tissue comprises restoring the function of the kidney or renal tissue.
Clause 8. The method of clause 4, wherein the tissue regeneration disease or disorder is a disease or disorder of the heart or cardiovascular system.
Clause 9. The method of clause 8, wherein the disease or disorder of the heart of cardiovascular system is selected from the group consisting of: pulmonary artery hypertension, acute myocardial infarction, chronic congestive heart failure, cardiomyopathy and coronary vasculopathy.
Clause 10. The method of clause 8, wherein treating the disease or disorder of the heart or cardiovascular system comprises restoring the function of the heart or cardiovascular system.
Clause 11. The method of clause 4, wherein the tissue regeneration disease or disorder is a disease or disorder of the liver or hepatic system.
Clause 12. The method of clause 11, wherein the disease or disorder of the liver or hepatic system is liver failure.
Clause 13. The method of clause 11, wherein treating the disease or disorder of the liver or hepatic system comprises restoring the function of the liver or hepatic system.
Clause 14. The method of clause 4, wherein the tissue regeneration disease or disorder is cancer.
Clause 15. The method of clause 14, wherein the cancer is selected from the group consisting of: breast cancer, prostate cancer and glioblastoma.
Clause 16. The method of clause 4, wherein the tissue degenerative disease is selected from the group consisting of renal disease, macular degeneration, degenerative joint disease, traumatic brain or spinal cord injury, stroke, atherosclerosis, arthritis, emphysema, osteoporosis, cardiomyopathy, cirrhosis, degenerative nerve disease, Holt-Oram disease, eye disease, diabetic nephropathy, degenerative bone disease, liver disease, periodontal disease, diabetes, cardiovascular disease, inflammatory disease, immune disease, skeletal disease, reproductive disease, haematopoietic disease, cellular damage due to ionizing radiation, cellular damage due to hypoxia and cancer.
Clause 17. The method of clause 16, wherein treating the tissue degenerative disease comprises restoring the function of the tissue.
Clause 18. The method of clause 4, wherein the tissue regeneration is selected from the group consisting of muscle, dendritic tissue, nerve, kidney, brain, bone, skin, lung, muscle, ovary, testes, heart, spleen, cartilage, nerve, peridontal, dentin, liver, vascular, connective, lymphatic, haematopoietic, and renal tissue.
Clause 19. A method of treating or preventing a disease or disorder associated with fibrosis in a subject, the method comprising administering to a subject in need of treatment at least one TDFRP in combination with an additional agent, wherein the additional agent is selected from the group consisting of: an inhibitor of angiotensin converting enzyme (ACE), a neprilysin inhibitor and an angiotensin receptor-neprilysin inhibitor, wherein the TDFRP and additional agent are administered in an amount effective to treat the fibrosis in the subject.
Clause 20. The method of clause 19, wherein the fibrosis is selected from the group consisting of: pulmonary fibrosis, renal fibrosis and hepatic fibrosis.
Clause 21. The method of clause 20, wherein the pulmonary fibrosis is idiopathic pulmonary fibrosis.
Clause 22. The method of clause 19, wherein the fibrosis is associated with a disease or condition selected from the group consisting of: atherosclerosis, cardiac failure, cardiac arrhythmia, myocardial infarction, peripheral vascular disease, diabetes, chronic renal disease, pulmonary fibrosis, liver failure, and Alzheimer's disease.
Clause 23. The method of clause 19, wherein the disease or condition is a chronic disease or condition.
Clause 24. The method of clause 16, wherein treating the pulmonary fibrosis, renal fibrosis or hepatic fibrosis comprises restoring the function of the pulmonary, renal or hepatic tissue.
Clause 25. The method of any one of clauses 1-23, wherein the TDFRP is a Multiple Domain TDFRP.
Clause 26. The method of any one of clauses 1-23, wherein the angiotensin converting enzyme (ACE) inhibitor is selected from the group consisting: of captopril, zofenopril, enalapril, ramipril, quinapril, perindopril, lisinopril, benazepril, imidapril, trandolapril, fosinopril, moexipril, cilazapril, spirapril, temocapril, alacepril, ceronapril, delepril, moveltipril, and combinations thereof.
Clause 27. The method of any one of clauses 1-23, wherein the neprilysin inhibitor is selected from the group consisting of: thiorphan, candoxatril, and candoxatrilat.
Clause 28. The method of any one of clauses 1-23, wherein the angiotensin receptor-neprilysin inhibitor is sacubitril/valsartan.
Clause 29. The method of any one of clauses 1-28, wherein the TDFRP and the additional agent are administered separately, sequentially and/or concurrently.
Clause 30. The method of any one of clauses 1-28, wherein the TDFRP is provided in the same composition as the additional agent.
Clause 31. The method of any one of clauses 1-28, wherein the TDFRP is provided in a separate composition as the additional agent.
Clause 32. The method of any one of clauses 1-28, wherein the TDFRP is administered by intravenous, intraperitoneal, subcutaneous, topical or other routes.
Clause 33. The method of any one of clauses 1-24, wherein the additional agent is administered by intravenous, intraperitoneal, subcutaneous, topical or other routes.
Clause 34. The method of any one of the clauses 1-33, further comprising the administration of a therapeutic agent selected from the group consisting of: anti-neoplastic agents, antibiotics, vaccines, immunosuppressive agents, anti-hypertensive agents and mediators of the hedgehog signaling pathway.
Clause 35. A method of increasing the serum half-life of a tissue differentiation factor related polypeptide (TDFRP) in a subject, the method comprising: administering to the subject at least one tissue differentiation factor related polypeptide (TDFRP) in combination with an additional agent, wherein the additional agent is selected from the group consisting of: an inhibitor of angiotensin converting enzyme (ACE), a neprilysin inhibitor and an angiotensin receptor-neprilysin inhibitor, wherein administration of the TDFRP and additional agent increases the serum half-life of the TDFRP compared to administration of the TDFRP alone.
Clause 36. The method of clause 35, wherein serum half-life is increased 2-fold or more when the TDFRP is administered with the additional agent.
Clause 37. The method of clause 35, wherein serum half-life is increased 5-fold or more when the TDFRP is administered with the additional agent.
Clause 38. The method of clause 35, wherein serum half-life is increased 10-fold or more when the TDFRP is administered with the additional agent.
Clause 39. The method of clause 35, wherein the subject has a tissue differentiation factor-associated disorder or disease.
Clause 40. The method of clause 35, wherein the subject has a disease or disorder associated with fibrosis.
Clause 41. A composition comprising at least one TDFRP and an additional agent, wherein the additional agent is selected from the group consisting of: an inhibitor of angiotensin converting enzyme (ACE), a neprilysin inhibitor and an angiotensin receptor-neprilysin inhibitor.
Clause 42. A pharmaceutical composition comprising the composition of clause 41 and a pharmaceutical excipient.
Clause 43. A kit comprising the pharmaceutical composition of clause 42 and instructions for use in treating or preventing a tissue differentiation factor-associated disorder or disease.
Clause 44. A kit comprising the pharmaceutical composition of clause 42, and instructions for use in treating or preventing fibrosis in a subject.

### REFERENCES

1. Agrotis et al., Biochem. Biophys. Res. Commun. 1996 Feb 15;219(2):613-8.
2. Aoki et al., J Cell Sci 2001 Apr;114(Pt. 8):1483-9.
3. Chien et al., J Periodontal Res. 1999 Aug;34(6):301-9.
4. De Winter et al., Exp Cell Res. 1996 May 1;224(2):323-34.
5. Dewulf et al., Endocrinology. 1995 Jun;136(6):2652-63.
7. Goggins et al., Cancer Res. 1998 Dec 1;58(23):5529-32.
8. Haaijman et al., Growth Factors. 2000;17(3):177-92.
9. Kawai et al., Biochem Biophys Res Commun. 2000 May 19;271(3):682-7.
10. ten Dijke et al., Science. 1994 Apr 1;264(5155):101-4.
11. ten Dijke et al., J Biol Chem 1994 Jun 24;269(25):16985-8.
12. ten Dijke et al., Oncogene 1993 Oct;8(10):2879-87.
13. Valcourt et al., J Biol Chem 2002 Sept 13;277(37):33545-58.
14. Abdalla et al., Eur J Hum Genet. 2003 Apr;11(4):279-87.
15. Agrotis et al., FEBS Lett 2000 Feb 4;467(1):128-32.
16. Akiyama et al., Cell Struct. Funct. 2000 Jun;25(3):195-204.
17. Ali et al., Pharmacology. 1998 Jul; 57(1):20-7.
18. Armes et al., Development. 1997 Oct;124(19):3797-804.
19. Aspenberg et al., Acta Orthop Scand. 2000 Dec;71(6):558-62.
20. Bobik et al., Circulation 1999 Jun 8;99(22):2883-91.
21. Choi et al., Am J Physiol 1997 Sept;273(3 pt.2):F386-95.
22. Chow et al., Mol Hum Reprod 2001 Nov;7(11):1047-56.
23. Goumans et al., Int J Dev Biol 2000 Apr;44(3):253-65.
24. Goumans et al., Differentiation 1998 Jul;63(3):101-13.
25. Inman et al., Mol Pharmacol 2002 Jul;62(1):65-74.
26. Gold et al., Am J Pathol 1997 Jan;150(1):209-22.
27. Kang et al., Biochem Mol Biol Int. 1996 Nov;40(5):993-1001.
28. Kim et al., Exp Cell Res 1998 May 25;241(1):151-60.
29. Laping et al., Mol Pharmacol 2002 Jul;62(1):58-64.
30. Larsson et al., EMBO J. 2001 Apr 2;20(7):1663-73.
31. Lui et al., Kidney Int. 1998 Mar;53(3):716-25.
32. Lui et al., Dev Dyn. 2000 Apr;217(4):343-60.
33. Liu et al., Zhonghua Xue Ye Xue Za Zhi. 2002 Oct;23(10):524-7.
34. Lui et al., FEBS Lett. 2002 May 22;519(1-3):93-8.
35. Lux et al., J Biol Chem. 1999 Apr 9;274(15):9984-92.
36. Machida et al., Eur J Endocrinol 2000 Nov;143(5):705-10.
37. Miettinen et al., J Cell Biol 1994 Dec;127(6 pt. 2):2021-36.
38. Mishina et al., Genes Dev. 1995 Dec 15;9(24):3027-37.
39. Mo et al., J Biol Chem 2002 Dec. 27;277(52):50788-94. Epub 2002 Oct. 18.
40. Morita et al., Brain Res Mol Brain Res 1996 Dec;42(2):263-71.
41. Panchenko et al., Am J Physiol 1996 Apr;270(4 pt 1):L547-58.
42. Piek et al., J Cell Sci 1999 Dec;12(pt 24):4557-68.
43. Ramp et al., Lab Invest. 1997 May;76(5):739-49.
44. Ryden et al., J Biol Chem 1996 Nov 29;271(48):30603-9.
45. Takumi et al., Exp Cell Res 1995 Jan;216(1):208-14.
46. Ward et al., Arterioscler Thromb Vasc Biol 1997 Nov;17(11):2461-70.
47. Ward et al., Arterioscler Thromb Vasc. Biol 2002 Jun 1;22(6):940-8.
48. Ward et al., FEBS Lett 1998 Jan 30;422(2): 197-200.
49. Ward et al., Atherosclerosis. 1998 Apr;137(2):267-75.
50. Wilson et al., Biol Reprod 2001 Apr;64(4):1225-35.
51. Wu et al., J Cell Physiol 1996 Aug;168(2):453-61.
52. Abe et al., J Bone Min. Res. 2000 Apr;15(4):663-73.
53. Adelman et al., Development. 2002 Jan;129(2):539-49.
54. Akiyama et al., Exp Cell Res 1997 Sep 15;235(2):362-9.
55. Ashique et al., Int J Dev Biol 2002 Mar;46(2):243-53.
56. Baur et al., Development 2000 Feb;127(3):605-19.
57. Beck et al., BMC Neurosci. 2001;2(1):12 Epub 2001 Sep 11.
58. Belecky-Adams et al., J Comp Neruol. 2001 Feb 19;430(4):562-72.
59. Botchkarev et al., FASEB J. 2001 Oct;15(12):2205-14.
60. Buurma et al., Eur J Oral Sci. 1999 Aug;107(4):282-9.
61. Chang et al., Dev Biol. 2002 Nov 15;251(2):380-94.
62. Cheifetz, Crit Rev Oral Biol Med. 1999;10(2):182-98.
63. Cheifetz, Eur J Oral Sci 1998 Jan;106 Suppl 1:174-8.
64. Coskun, et al., J Neurosci Res. 2002 Sep 15;69(6):795-802.
65. De Caestecker et al., Respir Res. 2001;2(4):193-7.
66. Deng et al., Zhonghua Wai Ke Za Zhi. 2002 Aug;40(8):592-5.
67. Ebisawa et al., J Cell Sci 1999 Oct;112 (Pt 20):3519-27.
68. Enomoto-Iwamoto et al., J Cell Biol 1998 Jan 26; 140(2):409-18.
69. Erickson et al., Reprod Biol Endocrinol. 2003 Feb 5;1(1):9 Epub 2003.
70. Erlacher et al., J Bone Miner Res 1998 Mar;13(3):383-92.
71. Franzen et al., Endocrinology. 1999 Sep;140(9):4300-10.
72. Fujii et al., Mol Biol Cell. 1999 Nov;10(11):3801-13.
73. Gouedard et al., J Biol Chem. 2000 Sep 8;275(36):27973-8.
74. Gould et al., Kidney Int. 2002 Jan;61(1):51-60.
75. Gu et al., Arch Oral Biol 1996 Oct;41(10):919-23.
76. Guo et al., Clin Orthop. 1999 Aug;(365):175-83.
77. Haaijman et al., Growth Factors. 2000;17(3):177-92.
78. Hanada et al., J Cell Biochem. 2001 Mar 26;81(2):284-94.
79. Hillmann et al., Biomaterials 2002 Mar;23(6):1461-9.
80. Hoshi et al., Bone. 1997 Aug;21(2):155-62.
81. Howe et al., Nat Genet 2001 Jun;28(2):184-7.
82. Ide et al., Cancer Res. 1997 Nov 15;57(22):5022-7.
83. Ide et al., Oncogene 1997 Mar 20;14(11):1377-82.
84. Ikeda et al., Dev Dyn 1996 Jul;206(3):318-29.
85. Ishidou et al., J Bone Miner Res 1995 Nov;10(11):1651-9.
86. Jazwinska et al., Cell. 1999 Feb 19;96(4):563-73.
87. Jikko et al., J Bone Miner Res 1999 Jul;14(7):1075-83.
88. Jin et al., Oral Oncol 2001 Apr;37(3) :225-33.
89. Kaneko et al., Bone. 2000 Oct ;27(4) :479-86.
90. Kawakami et al., Development 1996 Nov ;122(11) :3557-66.
91. Kim et al., Cancer Res 2000 Jun 1;60(11):2840-4.
92. Kirsch et al., EMBO J 2000 Jul 3;19(13):3314-24.
93. Kitten et al., J Cell Physiol 1999 Dec;181(3):410-5.
94. Kleeff et al, Gastroenterology. 1999 May;116(5):1202-16.
95. Kloen et al., J Bone Joint Surg. Am. 2002 Nov;84-A(11):1909-18.
96. Koenig et al., Mol Cell Biol 1994 Sep;14(9):5961-74.
97. Lecerf et al., Nucleic Acids Res 2001 Sep 1;29(17):E87-7.
98. Li et al., J Bone Miner Res 2001 Jun;16(6):1068-76.
99. Liu et al., Mol Cell Biol 1995 Jul;15(7):3479-86.
100. Liu et al., Dev Biol 2003 Apr 1;256(1):34-48.
101. Macias-Silva et al., J Biol Chem 1998 Oct 2;273(40):25628-36.
102. Marinova-Mutafchieva et al., Arthritis Rheum. 2002 Feb;46(2):507-13.
103. Martinez et al., Int J Dev Biol 2002;46(4):525-33.
104. Martinez et al., Exp Nephrol. 2001;9(6):372-9.
105. McNatty et al., Reprod Fertil Dev. 2001;13(7-8):549-55
106. McPherson et al., Dev Biol 2000 May 1-221(1):220-32.
107. Ming et al., Neuroscience 2002;114(4):849-57.
108. Miyazaki et al., J Clin Invest 2000 Apr;105(7):863-73.
109. Monsoro-Burq et al., Mech Dev. 2000 Oct;97(1-2):105-8.
110. Mori et al., Bone 1998 Feb;22(2):99-105.
111. Nakase et al., J Orthop Res 2001 Nov;19(6):1085-8.
112. Namiki et al., J Biol Chem. 1997 Aug 29;272(35):22046-52.
113. Natsume et al., J Biol Chem 1997 Apr 25;272(17):11353-40.
114. Nikaido et al., Mech Dev. 1999 Apr;82(1-2):219-22.
115. Nikaido et al., Development. 1999 Jan;126(1):181-90.
116. Nishihara et al., Biochem Biophys Res Commun. 2003 Feb 7;301(2):617-22.
117. Nishimura et al., J Biol Chem 1998 Jan 23;274(4):1872-9.
118. Nishitoh et al., J Biol Chem. 1996 Aug 30;271(35):21345-52.
119. Obata et al., Acta Ophthalmol Scand. 1999 Apr;77(2):151-6.
120. Onishi et al., Bone 1998 Jun;22(6):605-12.
121. Panchision et al., Genes Dev 2001 Aug 15 ;15(16) :2094-110.
122. Roelen et al., Int J Dev Biol 1997 Aug ;41(4) :541-9.
123. Sakou et al., J Bone Miner Res. 1999 Jul;14(7):1145-52.
124. Sanyal et al., J Orthop Res 2002 Jan;20(1):58-65.
125. Shimizu et al., Bone 1998 Aug;23(2):127-33.
126. Shukunami et al., FEBS Lett 2000 Mar 3;469(1):83-7.
127. Skillington et al., J Cell Biol 2002 Oct 14;159(1):135-46.
128. Soderstrom et al., Cell Tissue Res 1996 Nov;286(2):269-79.
129. Takae et al., Spine 1999 Jul 15;24(14):13897-401.
130. Takeda, Kokubyo Gakkai Zasshi, 1994 Dec;61(4):512-26.
131. Takeda et al., Biochem Biophys Res Commun 1994 Oct 14;204(1):203-9.
132. Tmaki et al., J Cell Physiol. 1998 Nov;177(2):355-63.
133. Toyono et al., J Dent Res. 1997 Sep;76(9):1555-60.
134. Toyono et al., Arch Oral Biol 1997 Jul;42(7):481-8.
135. van der Horst et al., Bone 2002 Dec;31(6):661-9.
136. Varley et al., Dev Biol 1998 Apr 1;196(1):107-18.
137. Volk et al., J Bone Miner Res 2000 Aug;15(8):1630-9.
138. Wada et al., Bone 1998 May;22(5):479-85.
139. Wollina et al., Acta Derm Venereol. 1999 May;79(3):183-6140.
140. Wu et al., J Cell Physiol 1996 Aug;168(2):453-61.
141. Yamada et al., Jpn J Opthalmol 1999 Jul-Aug;43(4):290-4.
142. Yamada et al., Br J Cancer 1996 Mar;73(5):624-9.
143. Yaoita et al., J Bone Miner Metab 2000;18(2):63-70.
144. Yazaki et al., Anticancer Res 1998 Jul-Aug;18(4A):2239-44.
145. Yeh et al., J Cell Physiol 2002 Mar;190(3):322-31.
146. Yeh et al., J Cell Physiol 2000 Oct;185(1):87-97.
147. Yeh et al., J Bone Miner Res 1998 Dec;13(12):1870-9.
148. Yi et al., Proc Natl Acad Sci USA 2001 Jul 3;98(14):7994-9 Epub 2001 Jun 19.
149. Yi et al., Development 2000 Feb;127(3):621-30.
150. Yonemori et al., Am J Pathol 1997 Apr;150(4):1335-47.
151. You et al., Invest Ophthalmol Vis Sci 1999 Feb;40(2):296-311.
152. Zehentner et al., J Bone Miner Res 1999 Oct;14(10):17.4-41.
153. Zhang et al., J Neurosci 1998 May 1;18(9):3314-26.
154. Zhao et al., J Bone Miner Res 2002 Aug;17(8):1441-51.
155. Zou et al., Genes Dev. 1997 Sep 1;11(17):2191-203.
156. Miyazono, Kohei; et al., Antibodies which bind specifically to activin receptor like kinases, Publ. No. 20020123139, US Patent Appl. No. 10/903,068, filed Sept. 5, 2002.
157. Caniggia, Isabella et al., Methods to diagnose a required regulation of trophoblast invasion, Publ. No. 20020110833, US Patent Appl. No. 10/028,158. filed August 15, 2002.
158. Plowman, Gregory et al., Diagnosis and treatment of ALK-7 related disorders, Publ. No. 20030073143, US Patent Appl. No. 10/069,228, filed April 17, 2003.
159. Rosenbaum, Jan Susan, Use of a BMP protein receptor complex for screening bone metabolism actives and cells cotransfected with a type II BMP receptor and a type I BMP receptor, 20030096296, US Patent Appl. No. 10/742,153, filed May 22, 2003.
160. Hughes, Paul E, et al., Methods of providing neuroprotection and/or neurorestoration via the neural activin type IIB receptor, Publ. No. 20030103959, US Patent Appl. No. 10/177,735, filed June 5, 2003.
161. Ibañez et al., Antibodies that specifically bind to ALK-7, a novel serine theronine kinase receptor, USSN 5811245, issued Sept. 22, 1998.
162. ten Dijke et al., Methods of antagonizing OP-1 binding to a cell surface receptor utilizing ALK polypeptides, USSN 5863738, issued Jan. 26, 1999.
163. Ibañez et al, Serine threonine kinase receptor, ALK-7, USSN 5891638, issued Apr. 6, 1999.
164. Ichijo et al., Method for identifying an OP-1 analog which binds an ALK-1 receptor, 5968752, Oct. 19, 1999.
165. Miyazono et al., Activin receptor-like kinases, ALK-3 and ALK-6, and nucleic acids encoding them, USSN 6207814, issued Mar. 27, 2001.
166. Rosenbaum, Use of a BMP protein receptor complex for screening bone metabolism actives and cells cotransfected with a type II BMP receptor and type I BMP receptor, USSN 6210899, issued Apr. 3, 2001.
167. Rosenbaum et al., CDNA encoding a BMP type II receptor, USSN 6306622, issued Oct. 23, 2001
168. Miyazono et al., Activin receptor-like kinases, proteins having serine threonine kinase domains and polynucleotides encoding same, 6316217, Nov. 13, 2001
169. Miyazono et al., Isolated nucleic acid molecules which encode activin-receptor like kinases, expression vectors and cells containing these, USSN 6331621, issued Dec. 18, 2001
170. Caniggia et al., Methods to diagnose a required regulation of trophoblast invasion, USSN 6376199, issued Apr. 23, 2002

## Claims

1. A composition for use in a method of treating or preventing a tissue differentiation factor-associated disorder or disease, wherein the composition comprises at least one tissue differentiation factor related polypeptide (TDFRP) in combination with an additional agent, wherein the additional agent is selected from the group consisting of: an inhibitor of angiotensin converting enzyme (ACE), a neprilysin inhibitor and an angiotensin receptor-neprilysin inhibitor, wherein the TDFRP and additional agent are administered in an amount effective to treat or prevent the tissue differentiation factor-associated disorder or disease in the subject.

2. The composition for use of claim 1, wherein the serum half-life of the TFDRP is increased when administered with the additional agent, compared to administration of the TDFRP alone; optionally, wherein the serum half-life is increased between about 5-fold to about 10-fold or more when the TDFRP is administered with the additional agent.

3. The composition for use of claim 1, wherein the tissue differentiation factor-associated disorder is selected from a tissue degenerative disease or a tissue regeneration disease;
optionally, wherein the tissue regeneration is selected from the group consisting of muscle, dendritic tissue, nerve, kidney, brain, bone, skin, lung, muscle, ovary, testes, heart, spleen, cartilage, nerve, peridontal, dentin, liver, vascular, connective, lymphatic, haematopoietic, and renal tissue; and/or
wherein the tissue regeneration disease or disorder is:
(i) a disease or disorder of the kidney or renal tissue; optionally, wherein the disease or disorder of the kidney or renal tissue is selected from the group consisting of: acute kidney injury and chronic kidney disease; or
(ii) a disease or disorder of the heart or cardiovascular system; optionally, wherein the disease or disorder of the heart of cardiovascular system is selected from the group consisting of: pulmonary artery hypertension, acute myocardial infarction, chronic congestive heart failure, cardiomyopathy and coronary vasculopathy; or
(iii) a disease or disorder of the liver or hepatic system; optionally, wherein the disease or disorder of the liver or hepatic system is liver failure; or
(iv) cancer; optionally, wherein the cancer is selected from the group consisting of: breast cancer, prostate cancer and glioblastoma.

4. The method of claim 3, wherein treating the disease or disorder of:
(i) the kidney or renal tissue comprises restoring the function of the kidney or renal tissue; or
(ii) the heart or cardiovascular system comprises restoring the function of the heart or cardiovascular system; or
(iii) the liver or hepatic system comprises restoring the function of the liver or hepatic system.

5. The composition for use of claim 3, wherein the tissue degenerative disease is selected from the group consisting of renal disease, macular degeneration, degenerative joint disease, traumatic brain or spinal cord injury, stroke, atherosclerosis, arthritis, emphysema, osteoporosis, cardiomyopathy, cirrhosis, degenerative nerve disease, Holt-Oram disease, eye disease, diabetic nephropathy, degenerative bone disease, liver disease, periodontal disease, diabetes, cardiovascular disease, inflammatory disease, immune disease, skeletal disease, reproductive disease, haematopoietic disease, cellular damage due to ionizing radiation, cellular damage due to hypoxia and cancer;
optionally, wherein treating the tissue degenerative disease comprises restoring the function of the tissue;
further optionally, wherein treating the pulmonary fibrosis, renal fibrosis or hepatic fibrosis comprises restoring the function of the pulmonary, renal or hepatic tissue.

6. A composition for use in a method of treating or preventing a disease or disorder associated with fibrosis in a subject, wherein the composition comprises at least one TDFRP in combination with an additional agent, wherein the additional agent is selected from the group consisting of: an inhibitor of angiotensin converting enzyme (ACE), a neprilysin inhibitor and an angiotensin receptor-neprilysin inhibitor, wherein the TDFRP and additional agent are administered in an amount effective to treat the fibrosis in the subject; optionally, wherein the disease or condition is a chronic disease or condition.

7. The composition for use of claim 6, wherein the fibrosis is selected from the group consisting of: pulmonary fibrosis, renal fibrosis and hepatic fibrosis;
optionally, wherein the pulmonary fibrosis is idiopathic pulmonary fibrosis;
further optionally, wherein the fibrosis is associated with a disease or condition selected from the group consisting of: atherosclerosis, cardiac failure, cardiac arrhythmia, myocardial infarction, peripheral vascular disease, diabetes, chronic renal disease, pulmonary fibrosis, liver failure, and Alzheimer's disease.

8. The composition for use of any one of claims 1-7, wherein the TDFRP is a Multiple Domain TDFRP; and/or
wherein the angiotensin converting enzyme (ACE) inhibitor is selected from the group consisting: of captopril, zofenopril, enalapril, ramipril, quinapril, perindopril, lisinopril, benazepril, imidapril, trandolapril, fosinopril, moexipril, cilazapril, spirapril, temocapril, alacepril, ceronapril, delepril, moveltipril, and combinations thereof; and/or
wherein the neprilysin inhibitor is selected from the group consisting of: thiorphan, candoxatril, and candoxatrilat; and/or
wherein the angiotensin receptor-neprilysin inhibitor is sacubitril/valsartan.

9. The composition for use of any one of claims 1-8, wherein the TDFRP and the additional agent are administered separately, sequentially and/or concurrently; optionally, wherein the TDFRP:
is provided in the same composition as the additional agent; and/or
is provided in a separate composition as the additional agent; and/or
is administered by intravenous, intraperitoneal, subcutaneous, topical or other routes;
further optionally, wherein the composition further comprises a therapeutic agent selected from the group consisting of: anti-neoplastic agents, antibiotics, vaccines, immunosuppressive agents, anti-hypertensive agents and mediators of the hedgehog signaling pathway.

10. A composition for use in a method of increasing the serum half-life of a tissue differentiation factor related polypeptide (TDFRP) in a subject, wherein the composition comprises at least one tissue differentiation factor related polypeptide (TDFRP) in combination with an additional agent, wherein the additional agent is selected from the group consisting of: an inhibitor of angiotensin converting enzyme (ACE), a neprilysin inhibitor and an angiotensin receptor-neprilysin inhibitor, wherein administration of the TDFRP and additional agent increases the serum half-life of the TDFRP compared to administration of the TDFRP alone.

11. The composition for use of claim 10, wherein serum half-life is increased 2-fold, 5-fold or 10-fold or more when the TDFRP is administered with the additional agent; optionally, wherein the subject has:
a tissue differentiation factor-associated disorder or disease; or
a disease or disorder associated with fibrosis.

12. The composition for use according to any of the preceding claims, wherein the composition is a pharmaceutical composition and a pharmaceutical excipient.

13. A composition comprising at least one TDFRP and an additional agent, wherein the additional agent is selected from the group consisting of: an inhibitor of angiotensin converting enzyme (ACE), a neprilysin inhibitor and an angiotensin receptor-neprilysin inhibitor.

14. The composition of claim 13, wherein the angiotensin converting enzyme (ACE) inhibitor is selected from the group consisting: of captopril, zofenopril, enalapril, enalaprilat, ramipril, quinapril, perindopril, lisinopril, benazepril, imidapril, trandolapril, fosinopril, moexipril, cilazapril, spirapril, temocapril, alacepril, ceronapril, delepril, moveltipril, and combinations thereof, wherein the neprilysin inhibitor is selected from the group consisting of: thiorphan, candoxatril, and candoxatrilat and wherein the angiotensin receptor- neprilysin inhibitor is sacubitril/valsartan.

15. The composition of claims 13 or 14, wherein the TDFRP is selected from the group consisting of: a Multiple Domain TDFRP, SEQ ID NO: 1, SEQ ID NO: 2 and SEQ ID NO: 3.
